# EUROPEAN PATENT SPECIFICATION

(11) **EP 3 411 058 B1**
(45) Date of publication and mention of the grant of the patent: **08.03.2023**
(21) Application number: 17747995.3
(22) Date of filing: 31.01.2017
(51) Int. Cl.: A61K 45/06, A61K 31/337, A61K 33/243, A61K 38/08, C07K 7/06, A61P 35/00, A61P 35/04

(54) **COMBINATION THERAPY WITH A6 AND PACLITAXEL FOR THE TREATMENT OF OVARIAN CANCER**
KOMBINATIONSTHERAPIE MIT A6 UND PACLITAXEL ZUR BEHANDLUNG DES OVARIALKARZINOMS
POLYTHÉRAPIE AVEC A6 ET PACLITAXEL POUR LE TRAITEMENT DU CANCER DES OVAIRES

(30) Priority: 02.02.2016 US 201662290306 P; 29.03.2016 US 201662314867 P; 29.07.2016 US 201662368964 P
(43) Date of publication of application: 12.12.2018
(73) Proprietor: Splash Pharmaceuticals, Inc., San Diego, CA 92121 (US)
(72) Inventor: FINLAYSON, Malcolm, San Diego, CA 92121 (US); NELSON, David, San Diego, CA 92121 (US)
(74) Representative: Held, Stephan
(86) International application number: PCT/US2017/015754
(87) International publication number: WO 2017/136312

(56) References cited:
- WO-A1-2011/022335
- WO-A1-2011/109678
- WO-A2-2010/102253
- US-A1- 2006 088 511
- US-A1- 2011 053 864

## Description

### CROSS-REFERNCE TO RELATED APPLICATIONS

This application claims priority to U.S. Provisional Patent Application No. 62/290,306, filed February 2, 2016, U.S. Provisional Patent Application No. 62/314,867, filed March 29, 2016, and to U.S. Provisional Patent Application No. 62/368,964, filed July 29, 2016.

### FIELD

The present invention relates to combinations of A6 peptide (SEQ ID NO:1 or SEQ ID NO: 2) and paclitaxel and the use of such combinations in the treatment of ovarian cancer.

### REFERENCE TO A SEQUENCE LISTING

This application includes a Sequence Listing created January 30, 2017, and being named "12963-021-228_SequenceListing.txt", which is 2,086 bytes in size. The material contained in the Sequence Listing is incorporated by reference in its entirety for all purposes.

### BACKGROUND

Mortality due to cancer is generally the result of metastasis of the primary tumor. Recurrence at distant sites following first-line therapy continues to be a major challenge. As a result, drugs that inhibit the metastatic process are of great interest. Metastasis and recurrence have been linked to a subpopulation of highly invasive tumorigenic cells that are characterized by the expression of CD44. There is a need for anti-cancer agents that can inhibit metastasis in ovarian cancer patients. Provided herein are solutions to these problems and other problems in the art.

### BRIEF SUMMARY

Provided, *inter alia,* herein are compositions comprising a CD44-modulating polypeptide described herein and paclitaxel described herein. The compositions can include a polypeptide of SEQ ID NO: 1 or SEQ ID NO:2 and paclitaxel. The compositions are useful for inhibiting metastasis of ovarian cancer by restoring sensitivity to paclitaxel as set forth herein.

In a first aspect is a method of treating ovarian cancer in a patient in need thereof by administering an effective amount of a CD44-modulating polypeptide described herein (e.g., SEQ ID NO: 1, SEQ ID NO: 2) in combination with an effective amount of an anti-cancer agent, e.g., paclitaxel. In one embodiment, the method includes treating ovarian cancer by administering a polypeptide having SEQ ID NO: 1 or SEQ ID NO:2 in combination with an effective amount of paclitaxel.

In another aspect is a pharmaceutical composition that includes a CD44-modulating polypeptide described herein (e.g., SEQ ID NO: 1, SEQ ID NO: 2), an anti-cancer agent (e.g., paclitaxel), and a pharmaceutically acceptable excipient. In still another aspect is a pharmaceutical composition that includes a polypeptide of SEQ ID NO: 1, paclitaxel, and a pharmaceutically acceptable excipient. In still another aspect is a pharmaceutical composition that includes a polypeptide of SEQ ID NO:2, paclitaxel, and a pharmaceutically acceptable excipient.

In any of the various aspects, the CD44-modulating polypeptides described herein (e.g., SEQ ID NO: 1, SEQ ID NO: 2) may be combined with paclitaxel as provided herein.

### BRIEF DESCRIPTION OF THE DRAWINGS

FIG. 1 illustrates that the connecting peptide domain is located between the N-terminal growth factor domain and the C-terminal catalytic domain of uPA
FIG. 2 illustrates that the polypeptide of SEQ ID NO: 1 shares sequence homology with a portion of the Link-Domain of CD44 (CD44 amino acid residues 120-NASAPPEE-127).

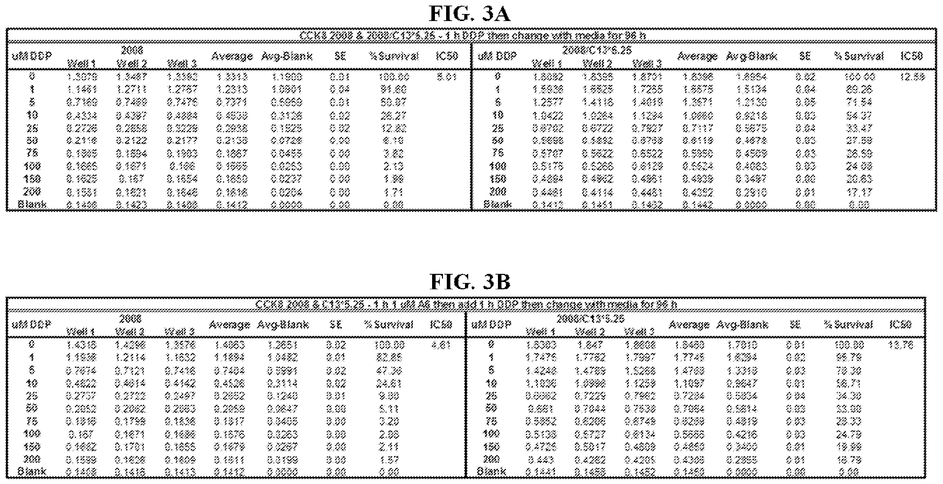

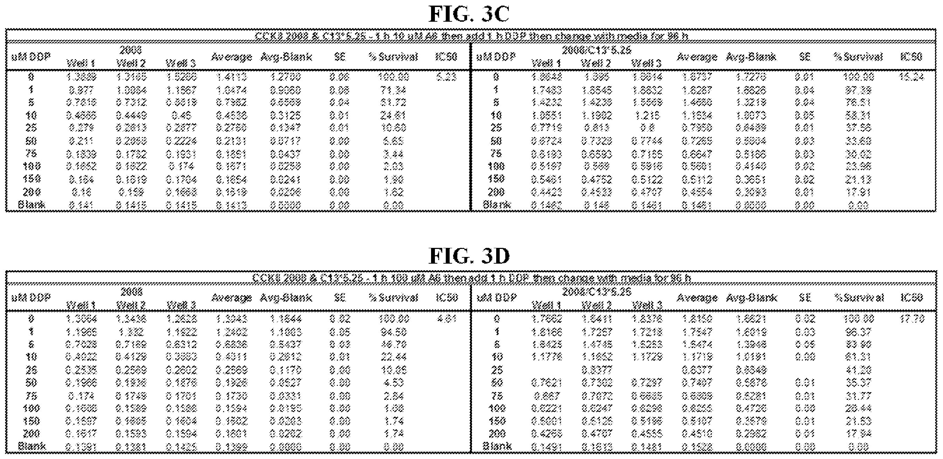

FIG. 3 illustrates testing of A6 plus Cisplatin (DDP) or Paclitaxel (PTX) in the B 16F 10-DsRed Cell Lung Metastasis Model. Tumor burden in the lungs was not significantly reduced by A6 in the presence or absence of paclitaxel. Cisplatin and lung metastasis models are embodiments not covered by the claims.
FIG. 4 illustrates testing of A6 plus Cisplatin (DDP) or Paclitaxel (PTX) in the B 16F 10-DsRed Cell Lung Metastasis Model. Tumor burden in the lungs was not significantly reduced by A6 in the presence or absence of cisplatin. Cisplatin and lung metastasis models are embodiments not covered by the claims.

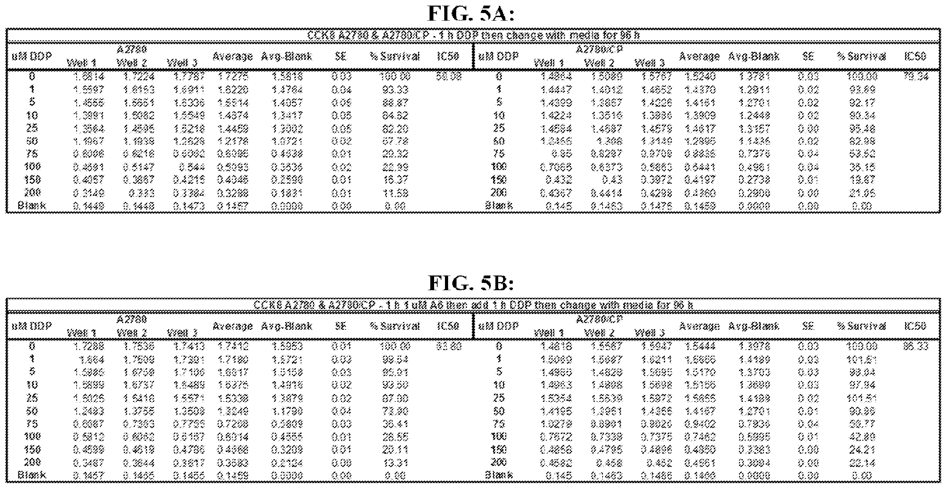

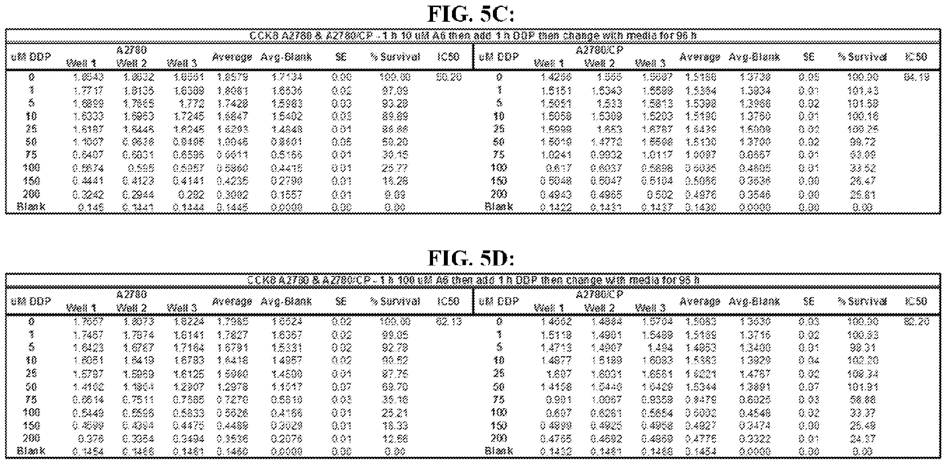

FIG. 5 illustrates testing of A6 plus Cisplatin (DDP) or Paclitaxel (PTX) in HEY (DDP sensitive) and HEY/C2 (DDP resistant) cells. Cisplatin is an embodiment not covered by the claims.

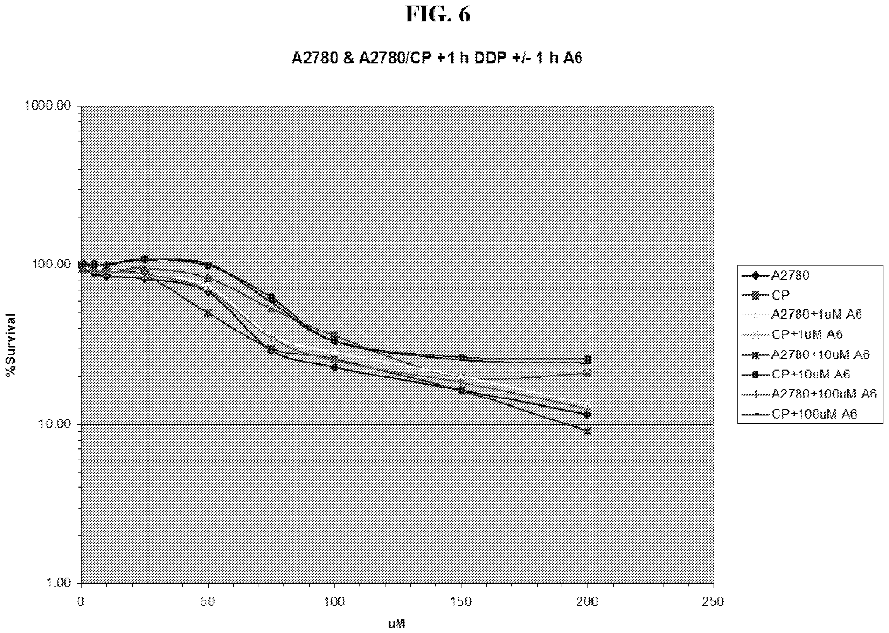

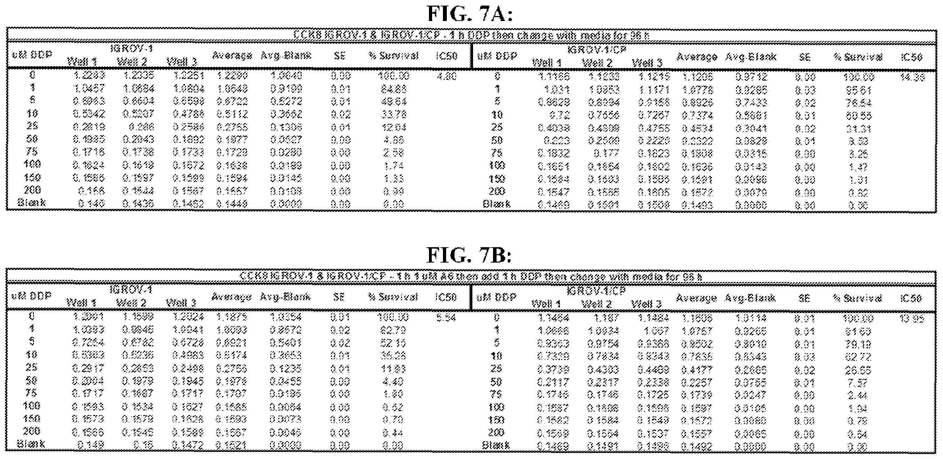

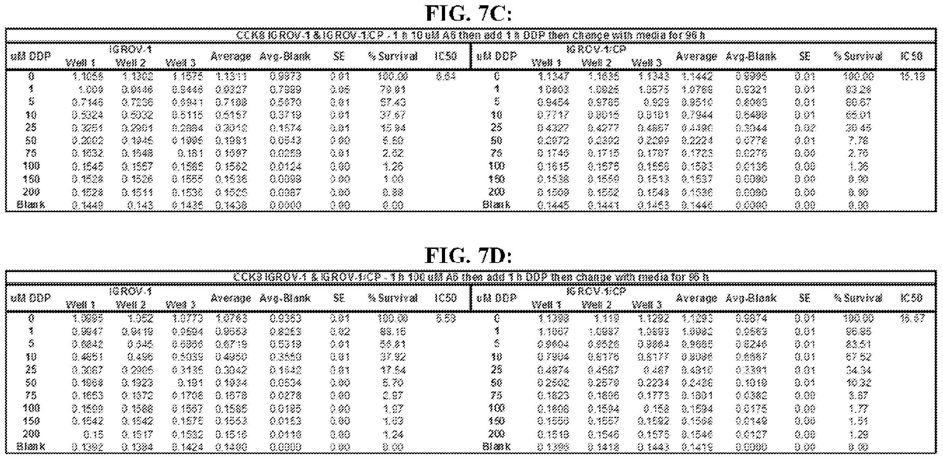

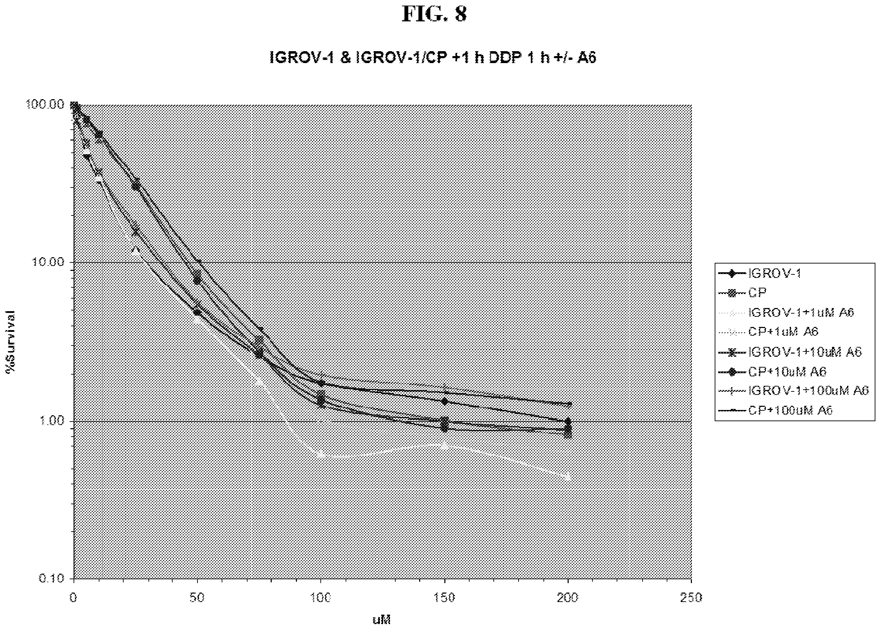

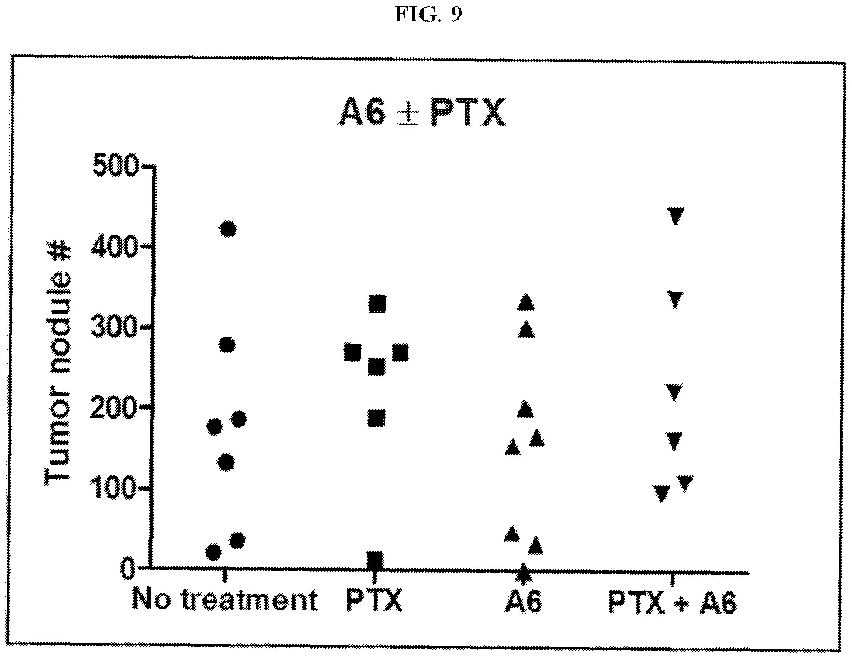

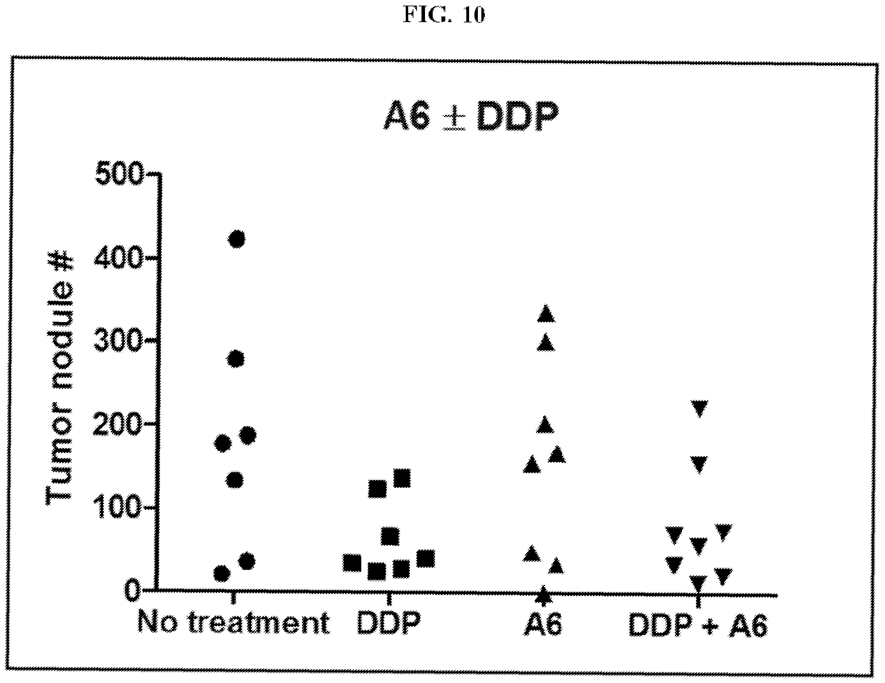

FIG. 6. Illustrates testing of A6 plus Cisplatin (DDP) or Paclitaxel (PTX) in HEY (DDP sensitive) and HEY/C2 (DDP resistant) cells. Cisplatin is an embodiment not covered by the claims.

### DETAILED DESCRIPTION

. Unless defined otherwise, all technical and scientific terms used herein have the same meaning as commonly understood by those of ordinary skill in the art to which the invention belongs. The chemical structures and formulae set forth herein are constructed according to the standard rules of chemical valency known in the chemical arts. Should a discrepancy exist between a depicted structure and a name given for that structure, the depicted structure is to be accorded more weight. Where the stereochemistry of a structure or a portion of a structure is not indicated in a depicted structure or a portion of the depicted structure, the depicted structure is to be interpreted as encompassing all of its possible stereoisomers.

Any methods, devices and materials similar or equivalent to those described herein can be used in the practice of this invention. The following definitions are provided to facilitate understanding of certain terms used frequently herein and are not meant to limit the scope of the present disclosure. In the event that there is a plurality of definitions for a term herein, those in this section prevail unless stated otherwise. Headings used herein are for organizational purposes only and in no way limit the invention described herein.

The terms "polypeptide" and "protein" are used interchangeably herein and refer to any molecule that includes at least 2 or more amino acids.

As used herein, "administering" and the like refer to the act physically delivering a composition or other therapy described herein into a subject by such routes as oral, mucosal, topical, transdermal, suppository, intravenous, parenteral, intraperitoneal, intramuscular, intralesional, intrathecal, intranasal or subcutaneous administration. Parenteral administration includes intravenous, intramuscular, intra-arterial, intradermal, subcutaneous, intraperitoneal, intraventricular, and intracranial administration. When a disease, disorder or condition, or a symptom thereof, is being treated, administration of the substance typically occurs after the onset of disease, disorder or condition or symptoms thereof. When a disease, disorder or condition, or symptoms thereof, are being prevented, administration of the substance typically occurs before the onset of the disease, disorder or condition or symptoms thereof.

The term "coadministration" refers to administration of two or more agents (*e.g.,* a polypeptide described herein and another active agent such as an anti-cancer agent described herein). The timing of coadministration depends in part on the combination and compositions or other therapies administered and can include administration at the same time, just prior to, or just after the administration of one or more additional therapies, for example cancer therapies such as chemotherapy, hormonal therapy, radiotherapy, or immunotherapy. Coadministration is meant to include simultaneous or sequential administration of a composition or therapy individually or in combination (more than one polypeptide described herein or an anti-cancer agent described herein as described herein). Coadministration can include administration of two or more agents where the agents are optionally combined with other active substances (*e.g.,* to reduce metabolic degradation). The polypeptides and anti-cancer agents described herein can be used in combination with one another, with other active agents known to be useful in treating a disease associated with cells expressing a particular kinase as described herein, or with adjunctive agents that cannot be effective alone, but can contribute to the efficacy of the active agent.

As used herein, the terms "subject" and "patient" are used interchangeably. As used herein, a subject can be a mammal such as a non-primate (*e.g.,* cows, pigs, horses, cats, dogs, rats, *etc.*) or a primate (*e.g.,* monkey and human). In specific embodiments, the subject is a human. In one embodiment, the subject is a mammal (*e.g.,* a human) having a disease, disorder or condition described herein. In another embodiment, the subject is a mammal (*e.g.,* a human) at risk of developing a disease, disorder or condition described herein. In certain instances the term patient refers to a human.

The terms "treating" or "treatment" refer to any indicia of success or amelioration of the progression, severity, and/or duration of a disease, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; or improving a patient's physical or mental well-being.

The term "cancer" refers to any physiological condition in mammals characterized by unregulated cell growth. Cancers described herein include solid tumors and hematological (blood) cancers. A "hematological cancer" refers to any blood borne cancer and includes, for example, myelomas, lymphomas and leukemias. A "solid tumor" or "tumor" refers to a lesion and neoplastic cell growth and proliferation, whether malignant or benign, and all pre-cancerous and cancerous cells and tissues resulting in abnormal tissue growth. "Neoplastic," as used herein, refers to any form of dysregulated or unregulated cell growth, whether malignant or benign, resulting in abnormal tissue growth. Not all cancers are embodiments covered by the claims.

An improvement in the cancer or cancer-related disease can be characterized as a complete or partial response. Complete response refers to an absence of clinically detectable disease with normalization of any previously abnormal radiographic studies, bone marrow, and cerebrospinal fluid (CSF) or abnormal monoclonal protein measurements. Partial response refers to at least about a 10%, 20%, 30%, 40%, 50%, 60%, 70%, 80%, or 90% decrease in all measurable tumor burden (*i.e.,* the number of malignant cells present in the subject, or the measured bulk of tumor masses or the quantity of abnormal monoclonal protein) in the absence of new lesions. The term "treatment" contemplates both a complete and a partial response.

A refractory, resistant, or persistent cancer refers to a circumstance where patients, even after intensive treatment, have residual cancer cells (*e.g.,* leukemia cells, lymphoma cells, circulating tumor cells or cancer stem cells) in their lymphatic system, blood and/or blood forming tissues (*e.g.,* marrow).

The terms "manage," "managing," and "management" refer to preventing or slowing the progression, spread or worsening of a disease or disorder, or of one or more symptoms thereof. In certain cases, the beneficial effects that a subject derives from a prophylactic or therapeutic agent do not result in a cure of the disease or disorder.

The term "preventing" refers to the treatment with or administration of a polypeptide or agent (e.g. anti-cancer agent described herein) provided herein, with or without other additional active agent (e.g. an anti-cancer agent), prior to the onset of symptoms, particularly to patients at risk of cancer and/or other disorders described herein. It should be understood that the polypeptides described herein can be coadministered with one or more anti-cancer agents described herein. The term prevention includes the inhibition or reduction of a symptom of the particular disease, as well as a reduced incidence of a symptom of the particular disease (e.g. by comparison to historical data for a given subject, or population data for similar subjects). Patients with familial history of a disease in particular are candidates for preventive regimens in certain embodiments. In addition, patients who have a history of recurring symptoms are also potential candidates for the prevention. In this regard, the term "prevention" may be interchangeably used with the term "prophylactic treatment."

A prophylactically effective amount of a polypeptide or agent (e.g. an anti-cancer agent described herein) means an amount of therapeutic agent, alone or in combination with other agents, which provides a prophylactic benefit in the inhibition or reduced incidence of a symptom of a disease or recurrence of a disease. The term prophylactically effective amount can encompass an amount that improves overall prophylaxis or enhances the prophylactic efficacy of another prophylactic agent.

The term "effective amount" as used herein refers to the amount of a therapy (*e.g.,* a composition provided herein) which is sufficient to reduce and/or ameliorate the severity and/or duration of a given disease, disorder or condition and/or a symptom related thereto. This term also encompasses an amount necessary for the reduction or amelioration of the advancement or progression of a given disease, disorder or condition, reduction or amelioration of the recurrence, development or onset of a given disease, disorder or condition, and/or to improve or enhance the prophylactic or therapeutic effect(s) of another therapy. In some embodiments, "effective amount" as used herein also refers to the amount of therapy provided herein to achieve a specified result.

As used herein, and unless otherwise specified, the term "therapeutically effective amount" of a polypeptide described herein or an anti-cancer agent described herein is an amount sufficient to provide a therapeutic benefit in the treatment or management of a cancer, or to delay or minimize one or more symptoms associated with the presence of the cancer. A therapeutically effective amount of a polypeptide described herein or an anti-cancer agent described herein means an amount of therapeutic agent, alone or in combination with other therapies, which provides a therapeutic benefit in the treatment or management of the cancer. The term "therapeutically effective amount" can encompass an amount that improves overall therapy, reduces or avoids symptoms or causes of the type of cancer, or enhances the therapeutic efficacy of another therapeutic agent.

A therapy is any protocol, method and/or agent that can be used in the prevention, management, treatment and/or amelioration of a given disease, disorder or condition. In certain embodiments, the terms "therapies" and "therapy" refer to a drug therapy, biological therapy, supportive therapy, radiation therapy, and/or other therapies useful in the prevention, management, treatment and/or amelioration of a given disease, disorder or condition known to one of skill in the art such as medical personnel. Not all therapies are embodiments covered by the claims.

A regimen is a protocol for dosing and timing the administration of one or more therapies (e.g., combinations described herein, another active agent such as for example an anti-cancer agent described herein) for treating a disease, disorder, or condition described herein. A regimen can include periods of active administration and periods of rest as known in the art. Active administration periods include administration of combinations/compositions described herein and the duration of time of efficacy of such combinations/compositions. Rest periods of regimens described herein include a period of time in which no agent (e.g., a polypeptide described herein or an anti-cancer agent described herein) is actively administered, and in certain instances, includes time periods where the efficacy of such agents can be minimal. Combination of active administration and rest in regimens described herein can increase the efficacy and/or duration of administration of the combinations and compositions described herein.

The term "pharmaceutically acceptable" as used herein refers to physiologically acceptable compounds, agents, or ingredients recognized by a regulatory agency of the Federal or state government, or another governmental agency with authorization for such approval, or and an agent listed in the U.S. Pharmacopeia, European Pharmacopeia or other generally recognized Pharmacopeia for use in animals, and more particularly in humans.

A "pharmaceutically acceptable excipient," refers to a substance that aids the administration of an active agent to a subject by for example modifying the stability of an active agent or modifying the absorption by a subject upon administration. A pharmaceutically acceptable excipient typically has no significant adverse toxicological effect on the patient. Examples of pharmaceutically acceptable excipients include, for example, water, NaCl (including salt solutions), normal saline solutions, sucrose, glucose, binders, fillers, disintegrants, lubricants, coatings, sweeteners, flavors, alcohols, oils, gelatins, carbohydrates such as amylose or starch, fatty acid esters, hydroxymethycellulose, polyvinyl pyrrolidine, and colors, and the like. One of skill in the art will recognize that other pharmaceutical excipients known in the art are useful in the present invention and include those listed in for example the Handbook of Pharmaceutical Excipients, Rowe R.C., Shesky P.J., and Quinn M.E., 6th Ed., The Pharmaceutical Press, RPS Publishing (2009). The terms binder, filler, disintegrant, and lubricant are used in accordance with the plain and ordinary meaning within the art.

In certain embodiments, a pharmaceutically acceptable excipient may be incompatible (*e.g.,* cross-reacts) with other excipients or active agents described herein. In some embodiments, magnesium stearate, croscarmellose sodium, lactose, excipients comprising Mg, Ca, K, Li, or Nucleic acid, acesulfame potassium, ammonium alginate, calcium acetate, calcium alginate, calcium carbonate, calcium chloride, calcium lactate, calcium phosphate, calcium silicate, calcium stearate, calcium sulfate, carboxymethylcellulose calcium, carboxymethylcellulose sodium, docusate sodium, glycine, kaolin, magnesium aluminum silicate, magnesium carbonate, magnesium oxide, magnesium silicate, magnesium trisilicate, polacrilin potassium, polymethacrylates, potassium alginate, potassium benzoate, potassium bicarbonate, potassium chloride, potassium citrate, sodium alginate, sodium benzoate, sodium chloride, sodium lauryl sulfate, sodium starch glycolate, sodium stearyl fumarate, sulfobutylether beta-cyclodextrin, sodium stearate, talc, or zinc stearate are incompatible in the dosage forms described herein.

The term "anti-cancer agent" is used in accordance with its plain ordinary meaning and refers to a composition having anti-neoplastic properties or the ability to inhibit the growth or proliferation of cells. In certain embodiments, an anti-cancer agent is a chemotherapeutic. In certain embodiments, an anti-cancer agent is an agent identified herein having utility in methods of treating cancer. In certain embodiments, an anti-cancer agent is an agent approved by the FDA or similar regulatory agency of a country other than the USA, for treating cancer.

The term "chemotherapeutic" or "chemotherapeutic agent" is used in accordance with its plain ordinary meaning and refers to a chemical composition or compound having anti-neoplastic properties or the ability to inhibit the growth or proliferation of cells. "Chemotherapy" or "cancer therapy" refers to a therapy or regimen that includes administration of a combination, chemotherapeutic, or anti-cancer agent described herein.

A "CD44-modulating polypeptide" refers to a polypeptide that binds to CD44 and modulates its activity (*e.g.,* signaling activity). A CD44-modulating polypeptide can be a polypeptide sequence described herein or, in some embodiments, an antibody that specifically binds to CD44 and inhibits its downstream signaling activity. In one embodiment, a CD44-modulating polypeptide can be a polypeptide sequence described herein or, in some embodiments, an antibody that disrupts or inhibits signaling activity of a CD44 dependent co-receptor. In certain instances the CD44 dependent co-receptor is a receptor tyrosine kinase (RTK) such as, for example, Met, Ran, or VEGFR. In still another embodiment a CD44-modulating polypeptide can be a polypeptide sequence described herein or, in some embodiments, an antibody that disrupts CD44 co-receptor function or association of a CD44 co-receptor with CD44 or another signaling protein. In one embodiment, a CD44-modulating polypeptide described herein binds to CD44 and inhibits CD44 signaling activity or association with one or more ABC transporters. The ABC transporter can be a multidrug resistant protein (e.g., MDR1). Exemplary CD-44 modulating polypeptides include polypeptides having homology to the CD44-v6 region of human CD44. Such peptides can include substitution variants, addition variants, or chemical derivatives thereof including peptidomimetics. In one embodiment, the CD44-modulating polypeptide described herein is a polypeptide having the amino acid sequence of Ac-KPSSPPEE-NH₂ (SEQ ID NO:1), Ac-NASAPPEE-NH₂ (SEQ ID NO:2), QETWFQNGWQGKNP (SEQ ID NO:3), KEKWFENEWQGKNP (SEQ ID NO:4), or KEQWFGNRWHEGYR (SEQ ID NO:5). Another CD44-modulating polypeptide can be QIRQQPRDPPTETLELEVSPDPAS (SEQ ID NO:6). Such exemplary peptides can include substitution variants, addition variants, or chemical derivatives thereof including peptidomimetics. Other exemplary CD44-modulating peptides include those set forth in U.S. Patent Nos. 5,994,309; 6,696,416; and 6,963,587 and U.S. Patent Application Publication No. US2009192085. SEQ ID NO: 3, SEQ ID NO: 4, SEQ ID NO: 5, and SEQ ID NO: 6 are embodiments not covered by the claims.

The term "peptidomimetic," as used herein, means a peptide-like molecule that has the activity of the peptide upon which it is structurally based. Such peptidomimetics include chemically modified peptides, peptide-like molecules containing non-naturally occurring amino acids, and peptoids, and have an activity such as the selective homing activity of the peptide upon which the peptidomimetic is derived (see, for example, Goodman and Ro, Peptidomimetics for Drug Design, in "Burger's Medicinal Chemistry and Drug Discovery" Vol. 1 (ed. M.E. Wolff; John Wiley & Sons (1995), pages 803-861).

The term "prodrug" refers to a compound or polypeptide that is made more active *in vivo* through metabolism of a precursor drug. CD44-modulating polypeptides described herein can exist as prodrugs, as described in, for example, Hydrolysis in Drug and Prodrug Metabolism: Chemistry, Biochemistry, and Enzymology (Testa, Bernard and Mayer, Joachim M. Wiley-VHCA, Zurich, Switzerland 2003). Prodrugs of the polypeptides described herein are structurally modified forms of the peptide that readily undergo chemical changes under physiological conditions to provide the active polypeptide. Additionally, prodrugs can be converted to the active polypeptide by chemical or biochemical methods in an *ex vivo* environment.

A PD-1 inhibitor refers to a moiety (*e.g.,* compound, nucleic acid, polypeptide, antibody) that decreases, inhibits, blocks, abrogates or interferes with the activity or expression of PD-1 (e.g., Programmed Cell Death Protein 1; PD-1 (CD279); GI: 145559515), including variants, isoforms, species homologs of human PD-1 (*e.g.,* mouse) and analogs that have at least one common epitope with PD-1. A PD-1 inhibitor includes molecules and macromolecules such as, for example, compounds, nucleic acids, polypeptides, antibodies, peptibodies, diabodies, minibodies, nanobodies, single-chain variable fragments (ScFv), and functional fragments or variants thereof. Thus, a PD-1 inhibitor as used herein refers to any moiety that antagonizes PD-1 activity or expression. PD-1 inhibitor efficacy can be measured, for example, by its inhibitor concentration at 50% (half-maximal inhibitor concentration or IC₅₀). PD-1 inhibitors include exemplary compounds and compositions described herein. A PD-1 antibody refers to a PD-1 inhibitor which is a monoclonal or polyclonal antibody as described herein.

The terms nivolumab, pembrolizumab, pidilizumab, AMP-224, REGN2810, PDR 001, and MEDI0680 are used in accordance with their plain and ordinary meaning as understood in the art.

Provided herein are methods comprising administering an effective amount of a CD44-modulating polypeptide. In another aspect provided herein is a method comprising administering an effective amount of a polypeptide comprising the amino acid sequence Ac-KPSSPPEE-NH₂ (SEQ ID NO: 1, "A6") or Ac-NASAPPEE-NH₂ (SEQ ID NO:2) in combination with an effective amount of an anti-cancer agent (e.g., paclitaxel). Amino acids of polypeptides described herein are numbered in reference from their N to C terminal (or its equivalent). For example, SEQ ID NO:1 can be numbered as followed: Ac-K¹P²S³S⁴P⁵P⁶E⁷E⁸-NH₂.

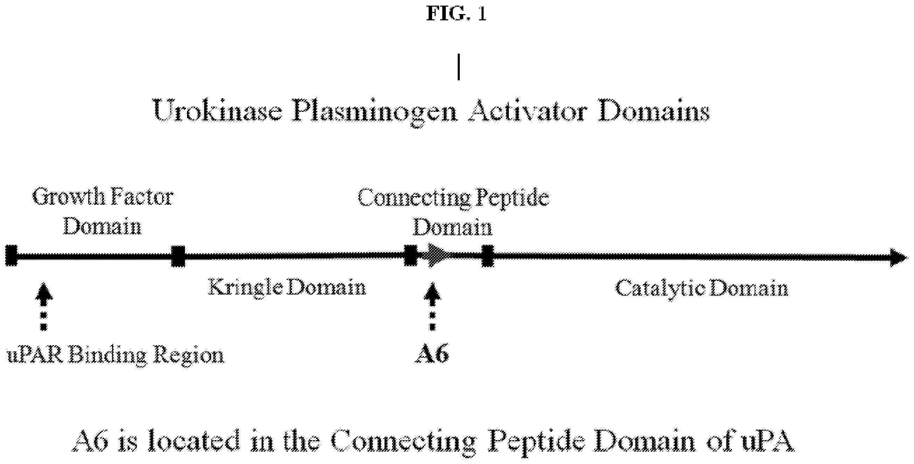

The polypeptide of SEQ ID NO: 1 can be a capped 8-amino acid peptide. The sequence of the polypeptide of SEQ ID NO:1 corresponds to amino acid residues 136-143 of the connecting peptide domain of human urokinase plasminogen activator (uPA). The connecting peptide domain is located between the N-terminal growth factor domain and the C-terminal catalytic domain of uPA (FIG. 1). The N-terminal growth factor domain of uPA binds to the uPA receptor (uPAR) to initiate the uPA/uPAR cascade. The binding of uPA to uPAR, can initiate a cascade of events leading to proteolysis, degradation of the extracellular matrix (ECM), cell migration, cell invasion, metastasis, and angiogenesis. Such events can promote cell death, including cell death of cancer cells in solid tumors and hematological cancers. The uPA system has been shown to play a role in the growth and spread of solid tumors. Levels of uPA and uPAR can correlate with clinical outcome in a variety of malignancies. In certain instances, the upregulation of the uPA system can be associated with poor prognosis. The inhibition of the uPA system can block critical processes (*e.g.,* cell migration, invasion, and angiogenesis) useful for a broad range of proliferative diseases.

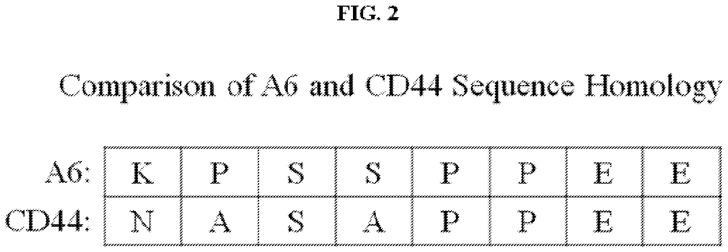

The polypeptide of SEQ ID NO: 1 shares sequence homology with a portion of the Link-Domain of CD44 (CD44 amino acid residues 120-NASAPPEE-127) (FIG. 2). The CD44 gene is encoded by 20 exons in the mouse and 19 exons in humans. There are 5 constant exons expressed at the 5' end, and 10 variant exons (mouse) or 9 variant exons (human) can be alternatively spliced within CD44 at an insertion site after the fifth constitutive exon, followed by the remaining constant exons at the 3' end. The smallest isoform of CD44 (CD44s) contains no variant exons. The largest isoform of CD44 (CD44v1-10) contains all of the variant exons. SEQ ID NO: 1 can be found nearly all CD44 isoforms, in part, because it is located within the first 5 non-variable exons of the isoform. SEQ ID NO:1 is located at the CD44 splice junction of exons 3 and 4.

The SEQ ID NO: 1 can include a substitution of K to A; P to A; S to A; or E to A. In some embodiments, the sequence of SEQ ID NO: 1 can be modified such that the CD44-modulating polypeptide includes a mutation of K¹ to A so long as the polypeptide retains activity about equal to or greater than the polypeptide of SEQ ID NO: 1. In another embodiment, SEQ ID NO: 1 can be modified to include mutation of P², P⁵, P⁶, or a combination thereof to A so long as the polypeptide retains activity about equal to or greater than the polypeptide of SEQ ID NO: 1. In certain embodiments, P² can be mutated to A. In certain embodiments, P⁵ can be mutated to A. In certain embodiments, P⁶ can be mutated to A. In another embodiment, S³, S⁴, or S³ and S⁴ can be mutated to A so long as the polypeptide retains activity about equal to or greater than the polypeptide of SEQ ID NO:1. In another embodiment, E⁷, E⁸, or E⁷ and E⁸ can be mutated to A so long as the polypeptide retains activity about equal to or greater than the polypeptide of SEQ ID NO: 1. It is to be understood that the above mutations can be combined in any manner to modify the polypeptide of SEQ ID NO: 1 so long as the polypeptide retains activity about equal to or greater than the polypeptide of SEQ ID NO: 1. Substitutions are embodiments not covered by the claims.

SEQ ID NO: 1 can include at least one glycosylation site. The glycosylation site can be an O-linked glycan on S³, S⁴, or S³ and S⁴ of SEQ ID NO: 1. In other instances, the glycosylation site can be present in any one Ser or Thr residue of SEQ ID NOs: 1-6. Glycosylations are embodiments not covered by the claims.

CD44-modulating polypeptides can have anti-migratory activity on cancer cells. In one embodiment, the CD44-modulating polypeptide described herein is a polypeptide comprising or consisting of SEQ ID NO: 1 or SEQ ID NO:2 and can have anti-migratory activity on cancer cells. A CD-44 modulating polypeptide can reduce the migratory activity of a cancer cell by at least 2, 3, 4, 5, 10, 15, 20, 25, 30, 35, 40, 45, 50, 55, 60, 65, 70, 75, 80, 85, 90, 95, or 100%. For example, the migratory activity of a cancer cell can be reduced by about: 1% to about 95%, 5% to about 95%, 10 to about 95%, 15% to about 95%, 25% to about 95%, 5% to about 100%, 10% to about 100%, 25% to about 100%; 10% to about 80%, or 10% to about 50%. In another example, the migratory activity can be reduced by at least 5, 10, 20, 25, 30, 40, or 50%. Such reductions can be measured against a control sample or, for example, a baseline sample taken from a subject prior to beginning any treatment described herein.

CD44-modulating polypeptides can have anti-invasive activity on cancer cells. In one embodiment, the CD44-modulating polypeptide described herein is a polypeptide comprising or consisting of SEQ ID NO: 1 or SEQ ID NO:2 and can have anti-invasive activity on cancer cells. A CD-44 modulating polypeptide can reduce the invasive activity of a cancer cell by at least 2, 3, 4, 5, 10, 15, 20, 25, 30, 35, 40, 45, 50, 55, 60, 65, 70, 75, 80, 85, 90, 95, or 100%. For example, the invasive activity of a cancer cell can be reduced by about: 1% to about 95%, 5% to about 95%, 10 to about 95%, 15% to about 95%, 25% to about 95%, 5% to about 100%, 10% to about 100%, 25% to about 100%; 10% to about 80%, or 10% to about 50%. In another example, the invasive activity can be reduced by at least 5, 10, 20, 25, 30, 40, or 50%. Such reductions can be measured against a control sample or, for example, a baseline sample taken from a subject prior to beginning any treatment described herein.

CD44-modulating polypeptides can have anti-metastatic activity on cancer cells. In one embodiment, the CD44-modulating polypeptide described herein is a polypeptide comprising or consisting of SEQ ID NO: 1 or SEQ ID NO:2 and can have anti-metastatic activity on cancer cells. A CD-44 modulating polypeptide can reduce the metastatic activity of a cancer cell by at least 2, 3, 4, 5, 10, 15, 20, 25, 30, 35, 40, 45, 50, 55, 60, 65, 70, 75, 80, 85, 90, 95, or 100%. For example, the metastatic activity of a cancer cell can be reduced by about: 1% to about 95%, 5% to about 95%, 10 to about 95%, 15% to about 95%, 25% to about 95%, 5% to about 100%, 10% to about 100%, 25% to about 100%; 10% to about 80%, or 10% to about 50%. In another example, the metastatic activity can be reduced by at least 5, 10, 20, 25, 30, 40, or 50%. Such reductions can be measured against a control sample or, for example, a baseline sample taken from a subject prior to beginning any treatment described herein.

The present invention includes embodiments where a CD44-modulating polypeptide described herein establishes, restores or enhances the anti-cancer activity of the anti-cancer agent (e.g., paclitaxel) in treating ovarian cancer that is resistant or refractory to the treatment. In one example, a CD44-modulating polypeptide establishes anti-cancer activity (e.g., creates efficacy of the anti-cancer agent in treating a cancer) of the anti-cancer agent (e.g., paclitaxel) in the treatment of ovarian cancer. In another example, a CD44-modulating polypeptide restores the anti-cancer activity of the anti-cancer agent (e.g., paclitaxel). In still another example, a combination therapy of a CD44-modulating polypeptide described herein and the anti-cancer agent (e.g., paclitaxel) establishes, restores, or enhances activity of a CD44-modulating polypeptide.

The ovarian cancer can optionally be resistant or refractory to a plurality of anti-cancer agents (e.g. two or more anti-cancer agents) and/or a plurality of radiation therapies. In one example the ovarian cancer can also be resistant, refractory, or non-responsive to treatment with a CD44-modulating polypeptide described herein. A patient may be administered a combination of a CD44-modulating polypeptide described herein and an anti-cancer agent (e.g., paclitaxel) where the ovarian cancer treated is resistant, refractory, or non-responsive to one of or both the CD44-modulating polypeptide and the anti-cancer agent (e.g., paclitaxel). In one example, the ovarian cancer can be resistant, refractory, or non-responsive to treatment with the anti-cancer agent (e.g., paclitaxel). Administration of the combination of the CD44-modulating polypeptide and anti-cancer agent(s) (e.g., paclitaxel) surprisingly can restore or enhance the activity of the anti-cancer agent (e.g., paclitaxel) against the refractory, resistant, or non-responsive ovarian cancer. Administration of the combination of the CD44-modulating polypeptide and anti-cancer agent(s) (e.g., paclitaxel) surprisingly can restore or enhance the activity of the CD44-modulating polypeptide against the refractory, resistant, or non-responsive ovarian cancer. Administration of the combination of the CD44-modulating polypeptide and anti-cancer agent(s) (e.g., paclitaxel) surprisingly can restore or enhance the activity of the CD44-modulating polypeptide and the anti-cancer agent (e.g., paclitaxel) against the refractory, resistant, or non-responsive ovarian cancer.

In one example, a CD44-modulating polypeptide described herein does not have activity against an ovarian cancer described herein when administered alone. In one embodiment, where a CD44-modulating polypeptide described herein does not have activity against an ovarian cancer described herein when administered alone, its activity can be established or restored when administered in combination with an anti-cancer agent described herein (e.g., paclitaxel). In another example, a CD44-modulating polypeptide described herein has minimal activity against an ovarian cancer described herein (e.g., insufficient anti-cancer activity to treat an ovarian cancer described herein) when administered alone. In one embodiment, where a CD44-modulating polypeptide described herein has minimal activity against an ovarian cancer described herein, its activity can be enhanced when administered in combination with an anti-cancer agent (e.g., paclitaxel) described herein.

In another example, an anti-cancer agent (e.g., paclitaxel) described herein does not have activity against an ovarian cancer described herein when administered alone (or in combination with another anti-cancer agent). In one example, where an anti-cancer agent described herein (e.g., paclitaxel) does not have activity against an ovarian cancer described herein when administered alone, its activity can be restored when administered in combination with CD44-modulating polypeptide described herein. In another example, an anti-cancer agent described herein (e.g., paclitaxel) has minimal activity against an ovarian cancer described herein (e.g., insufficient anti-cancer activity to treat an ovarian cancer described herein) when administered alone. In one example, where an anti-cancer agent described herein (e.g., paclitaxel) has minimal activity against an ovarian cancer described herein, its activity can be enhanced when administered in combination with a CD44-modulating polypeptide described herein. In another example, an anti-cancer agent (e.g., paclitaxel) can lose its anti-cancer activity over the course of treatment due to, for example, progression of resistance or refraction in the ovarian cancer treated. In one example, the loss of anti-cancer agent (e.g., paclitaxel) activity can be slowed, stopped, or reversed (e.g. enhanced activity) when the patient is administered the anti-cancer agent (e.g., paclitaxel) in combination with a CD44-modulating polypeptide described herein.

In certain instances, the methods above include administration of a polypeptide comprising or consisting of SEQ ID NO: 1 or SEQ ID NO:2. In one embodiment, the methods described herein include administration of a polypeptide of SEQ ID NO: 1.

The anti-cancer agent can be paclitaxel.

In certain instances, the CD44-modulating polypeptide does not have anti-cancer activity against an ovarian cancer described herein when administered alone. In one example, a polypeptide of SEQ ID NO: 1 or a polypeptide of SEQ ID NO:2 does not have anti-cancer activity against a cancer described herein when administered alone. In one embodiment, such a polypeptide (e.g., SEQ ID NO: 1, SEQ ID NO: 2) not having anti-cancer activity can restore or enhance the activity of a co-administered anti-cancer agent (e.g., paclitaxel) described herein.

In one embodiment, coadministration of a polypeptide of SEQ ID NO:1 or a polypeptide of SEQ ID NO:2, having known inactivity against an ovarian cancer, with an anti-cancer agent restores or enhances the anti-cancer activity of the polypeptide of SEQ ID NO: 1 or a polypeptide of SEQ ID NO:2. In another embodiment, coadministration of a polypeptide of SEQ ID NO: 1 or a polypeptide of SEQ ID NO:2, having known inactivity against an ovarian cancer, with an anti-cancer agent restores or enhances the anti-cancer activity of the polypeptide of SEQ ID NO:1 or a polypeptide of SEQ ID NO:2.

In another embodiment, coadministration of a polypeptide of SEQ ID NO:1 or SEQ ID NO:2, having known inactivity against a specific cancer, with an anti-cancer agent (e.g,. paclitaxel) to a patient having an ovarian cancer with resistance to the cancer agent (e.g,. paclitaxel), restores or enhances the anti-cancer activity of the polypeptide of SEQ ID NO:1 or SEQ ID NO:2 and/or the anti-cancer agent (e.g., paclitaxel). In one embodiment, the combination of CD44-modulating polypeptide described herein with an anti-cancer agent described herein (e.g,. paclitaxel) is useful in the treatment of ovarian cancers resistant to one or more anti-cancer agents (e.g,. paclitaxel) and/or a CD44-modulating polypeptide.

Exemplary anti-cancer agents that are not covered by the claims include but are not limited to: ABRAXANE; abiraterone; ace-11; aclarubicin; acivicin; acodazole hydrochloride; acronine; actinomycin; acylfulvene; adecypenol; adozelesin; adriamycin; aldesleukin; all trans-retinoic acid (ATRA); altretamine; ambamustine; ambomycin; ametantrone acetate; amidox; amifostine; aminoglutethimide; aminolevulinic acid; amrubicin; amsacrine; anagrelide; anastrozole; andrographolide; antarelix; anthramycin; aphidicolin glycinate; apurinic acid; ara-CDP-DL-PTBA; arginine deaminase; ARRY-162; ARRY-300; ARRY-142266; AS703026; asparaginase; asperlin; asulacrine; atamestane; atrimustine; AVASTIN; axinastatin 1; axinastatin 2; axinastatin 3; azasetron; azatoxin; azatyrosine; azacitidine; AZD8330; azetepa; azotomycin; balanol; batimastat; BAY 11-7082; BAY 43-9006; BAY 869766; bendamustine; benzochlorins; benzodepa; benzoylstaurosporine; beta-alethine; betaclamycin B; betulinic acid; b-FGF inhibitor; bicalutamide; bisantrene; bisaziridinylspermine; bisnafide; bisnafide dimesylate; bistratene A; bisantrene hydrochloride; bleomycin; bleomycin sulfate; busulfan; bizelesin; breflate; bortezomib; brequinar sodium; bropirimine; budotitane; buthionine sulfoximine; bryostatin; cactinomycin; calusterone; calcipotriol; calphostin C; camptothecin derivatives; capecitabine; carboxamide-amino-triazole; carboxyamidotriazole; CaRest M3; CARN 700; caracemide; carbetimer; carboplatin; carmustine; carubicin hydrochloride; carzelesin; castanospermine; cecropin B; cedefingol; celecoxib; cetrorelix; chlorins; chloroquinoxaline sulfonamide; cicaprost; chlorambucil; Chlorofusin; cirolemycin; cisplatin; CI-1040; cis-porphyrin; cladribine; clomifene analogues; clotrimazole; collismycin A; collismycin B; combretastatin A4; combretastatin analogue; conagenin; crambescidin 816; crisnatol; crisnatol mesylate; cryptophycin 8; cryptophycin A derivatives; curacin A; cyclopentanthraquinones; cycloplatam; cypemycin; cyclophosphamide; cytarabine; cytarabine ocfosfate; cytolytic factor; cytostatin; dacarbazine; dactinomycin; daunorubicin; daunorubicin hydrochloride; decarbazine; dacliximab; dasatinib; decitabine; dehydrodidemnin B; deslorelin; dexamethasone; dexifosfamide; dexrazoxane; dexverapamil; dexormaplatin; dezaguanine; dezaguanine mesylate; diaziquone; didemnin B; didox; diethylnorspermine; dihydro 5 azacytidine; dihydrotaxol; 9-dioxamycin; diphenyl spiromustine; docosanol; dolasetron; docetaxel; doxorubicin; doxorubicin hydrochloride; doxifluridine; droloxifene; droloxifene citrate; dromostanolone propionate; dronabinol; duazomycin; duocarmycin SA; ebselen; ecomustine; edelfosine; edrecolomab; edatrexate; eflomithine hydrochloride; eflornithine; elemene; emitefur; elsamitrucin; enloplatin; enpromate; epipropidine; epirubicin; epirubicin hydrochloride; epristeride; erbulozole; eribulin; esorubicin hydrochloride; estramustine; estramustine phosphate sodium; etanidazole; etoposide; etoposide phosphate; etoprine; exemestane; fadrozole; fadrozole hydrochloride; fazarabine; fenretinide; filgrastim; finasteride; flavopiridol; flezelastine; fluasterone; floxuridine; fludarabine phosphate; fludarabine; fluorodaunorubicin hydrochloride; forfenimex; formestane; fluorouracil; floxouridine; flurocitabine; fosquidone; fostriecin sodium; fostriecin; fotemustine; gadolinium texaphyrin; gallium nitrate; galocitabine; ganirelix; gelatinase inhibitors; gemcitabine; geldanamycin; gossyphol; GDC-0973; GSK1120212/trametinib; herceptin; hydroxyurea; hepsulfam; heregulin; hexamethylene bisacetamide; hypericin; ibandronic acid; ibrutinib; idarubicin; idarubicin hydrochloride; ifosfamide; canfosfamide; ilmofosine; iproplatin; idoxifene; idramantone; ilmofosine; ilomastat; imidazoacridones; imatinib (e.g., GLEEVEC); imiquimod; iniparib (BSI 201); iobenguane; iododoxorubicin; ipomeanol; irinotecan; irinotecan hydrochloride; irsogladine; isobengazole; isohomohalicondrin B; itasetron; iimofosine; interleukin IL-2 (including recombinant interleukin II; or rlL.sub.2); interferon alfa-2a; interferon alfa-2b; interferon alfa-n1; interferon alfa-n3; interferon beta-1a; interferon gamma-1b; jasplakinolide; kahalalide F; lamellarin N triacetate; lanreotide; leinamycin; lenograstim; lentinan sulfate; leptolstatin; letrozole; leuprorelin; levamisole; lenalidomide; lenvatinib; liarozole; lissoclinamide 7; lobaplatin; lombricine; lometrexol; lonidamine; losoxantrone; lovastatin; loxoribine; lurtotecan; lutetium texaphyrin; lysofylline; lanreotide acetate; lapatinib; letrozole; leucovorin; leuprolide acetate; liarozole hydrochloride; lometrexol sodium; lomustine; losoxantrone hydrochloride; pomalidomide; LY294002; maitansine; mannostatin A; marimastat; masoprocol; maspin; matrilysin inhibitors; menogaril; merbarone; meterelin; methioninase; metoclopramide; MIF inhibitor; mifepristone; miltefosine; mirimostim; mitoguazone; mitolactol; mitonafide; mitoxantrone; mofarotene; molgramostim; mopidamol; mycaperoxide B; myriaporone; maytansine; mechlorethamine hydrochloride; megestrol acetate; melengestrol acetate; melphalan; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nafarelin; nagrestip; napavin; naphterpin; nartograstim; nedaplatin; nemorubicin; neridronic acid; nilutamide; nisamycin; nitric oxide modulators; nitroxide antioxidant; nitrullyn; nocodazole; nogalamycin; oblimersen (GENASENSE); octreotide; okicenone; olaparib (LYNPARZA); oligonucleotides; onapristone; ondansetron; oracin; oral cytokine inducer; ormaplatin; oxisuran; oxaloplatin; osaterone; oxaliplatin; oxaunomycin; palauamine; palmitoylrhizoxin; pamidronic acid; panaxytriol; panomifene; parabactin; PARP (polyADP ribose polymerase) inhibitors; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; porfiromycin; prednisone; prostaglandin J2; pyrazoloacridine; PD035901; PD184352; PD318026; PD98059; peliomycin; pentamustine; peplomycin sulfate; PKC412; pipobroman; piposulfan; piroxantrone hydrochloride; plicamycin; plomestane; podophyllotoxin; polyphenol E; porfimer sodium; porfiromycin; prednimustine; procarbazine; procarbazine hydrochloride; puromycin; puromycin hydrochloride; pyrazofurin; raltitrexed; ramosetron; retelliptine demethylated; rhizoxin; rituximab; RII retinamide; rogletimide; rohitukine; romurtide; roquinimex; rubiginone B1; ruboxyl; riboprine; romidepsin; rucaparib; safingol; safingol hydrochloride; saintopin; sarcophytol A; sargramostim; semustine; sizofiran; sobuzoxane; sodium borocaptate; sodium phenylacetate; solverol; sonermin; sorafenib; sunitinib; sparfosic acid; spicamycin D; spiromustine; splenopentin; spongistatin 1; Spongistatin 2; Spongistatin 3; Spongistatin 4; Spongistatin 5; Spongistatin 6; Spongistatin 7; Spongistatin 8; and Spongistatin 9; squalamine; stipiamide; stromelysin inhibitors; sulfinosine; suradista; suramin; swainsonine; SB239063; selumetinib/AZD6244; simtrazene; SP600125; sparfosate sodium; sparsomycin; spirogermanium hydrochloride; spiroplatin; streptonigrin; streptozocin; sulofenur; tallimustine; tamoxifen methiodide; talazoparib (BMN 673); tauromustine; tazarotene; tecogalan sodium; tegafur; tellurapyrylium; temoporfin; temozolomide; teniposide; tetrachlorodecaoxide; tetrazomine; thaliblastine; thiocoraline; thrombopoietin; thymalfasin; thymopoietin receptor agonist; thymotrinan; tirapazamine; titanocene bichloride; topsentin; toremifene; tretinoin; triacetyluridine; triciribine; trimetrexate; triptorelin; tropisetron; turosteride; tyrphostins; talisomycin; TAK-733; taxotere; tegafur; teloxantrone hydrochloride; teroxirone; testolactone; thiamiprine; thioguanine; thiotepa; tiazofurin; tirapazamine; toremifene citrate; trastuzumab; trestolone acetate; triciribine phosphate; trimetrexate; trimetrexate glucuronate; triptorelin; tubulozole hydrochloride; tumor necrosis factor-related apoptosis-inducing ligand (TRAIL); UBC inhibitors; ubenimex; U0126; uracil mustard; uredepa; vapreotide; variolin B; velaresol; veliparib (ABT-888); veramine; verteporfin; vinorelbine; vinxaltine; vitaxin; vinblastine; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; wortmannin; XI,518; zanoterone; zeniplatin; zilascorb; zinostatin stimalamer; zinostatin; and zorubicin hydrochloride.

Other exemplary anti-cancer agents not covered by th claims include Erbulozole (*e.g.,* R-55104); Dolastatin 10 (*e.g.,* DLS-10 and NSC-376128); Mivobulin isethionate (*e.g.,* CI-980); NSC-639829; Discodermolide (*e.g.,* NVP-XX-A-296); ABT-751 (Abbott; *e.g.,* E-7010); Altorhyrtin A; Altorhyrtin C; Cemadotin hydrochloride (*e.g.,* LU-103793 and NSC-D-669356); CEP 9722; Epothilone A; Epothilone B; Epothilone C; Epothilone D; Epothilone E; Epothilone F; Epothilone B N-oxide; Epothilone AN-oxide; 16-aza-epothilone B; 21-aminoepothilone B; 21-hydroxyepothilone D; 26-fluoroepothilone; Auristatin PE (*e.g.,* NSC-654663); Soblidotin (*e.g.,* TZT-1027); LS-4559-P (Pharmacia; *e.g.,* LS-4577); LS-4578 (Pharmacia; *e.g.,* LS-477-P); LS-4477 (Pharmacia); LS-4559 (Pharmacia); RPR-112378 (Aventis); DZ-3358 (Daiichi); FR-182877 (Fujisawa; *e.g.,* WS-9265B); GS-164 (Takeda); GS-198 (Takeda); KAR-2 (Hungarian Academy of Sciences); BSF-223651 (BASF; *e.g.,* ILX-651 and LU-223651); SAH-49960 (Lilly/Novartis); SDZ-268970 (Lilly/Novartis); AM-97 (Armad/Kyowa Hakko); AM-132 (Armad); AM-138 (Armad/Kyowa Hakko); IDN-5005 (Indena); Cryptophycin 52 *(e.g.,* LY-355703); AC-7739 (Ajinomoto; *e.g.,* AVE-8063A and CS-39.HCl); AC-7700 (Ajinomoto; *e.g.,* AVE-8062; AVE-8062A; CS-39-L-Ser.HCl; and RPR-258062A); Vitilevuamide; Tubulysin A; Canadensol; CA-170 (Curis, Inc.); Centaureidin (*e.g.,* NSC-106969); T-138067 (Tularik; *e.g.,* T-67; TL-13 8067 and TI-138067); COBRA-1 (Parker Hughes Institute; *e.g.,* DDE-261 and WHI-261); H10 (Kansas State University); H16 (Kansas State University); Oncocidin A1 (*e.g.,* BTO-956 and DIME); DDE-313 (Parker Hughes Institute); Fijianolide B; Laulimalide; SPA-2 (Parker Hughes Institute); SPA-1 (Parker Hughes Institute; *e.g.,* SPIKET-P); 3-IAABU (Cytoskeleton/Mt. Sinai School of Medicine; *e.g.,* MF-569); Narcosine (*e.g.,* NSC-5366); Nascapine; D-24851 (Asta Medica); A-105972 (Abbott); Hemiasterlin; 3-BAABU (Cytoskeleton/Mt. Sinai School of Medicine; *e.g.,* MF-191); TMPN (Arizona State University); Vanadocene acetylacetonate; T-138026 (Tularik); Monsatrol; lnanocine (*e.g.,* NSC-698666); 3-IAABE (Cytoskeleton/Mt. Sinai School of Medicine); A-204197 (Abbott); T-607 (Tuiarik; e.g., T-900607); RPR-115781 (Aventis); Eleutherobins (*e.g.,* Desmethyleleutherobin; Desaetyleleutherobin; lsoeleutherobin A; and Z-Eleutherobin); Caribaeoside; Caribaeolin; Halichondrin B; D-64131 (Asta Medica); D-68144 (Asta Medica); Diazonamide A; A-293620 (Abbott); NPI-2350 (Nereus); Taccalonolide A; TUB-245 (Aventis); A-259754 (Abbott); Diozostatin; (-)-Phenylahistin (*e.g.,* NSCL-96F037); D-62638 (Asta Medica); D-62636 (Asta Medica); Myoseverin B; D-43411 (Zentaris; e.g., D-81862); A-289099 (Abbott); A-318315 (Abbott); HTI-286 (*e.g.,* SPA-110; trifluoroacetate salt) (Wyeth); D-82317 (Zentaris); D-82318 (Zentaris); SC-12983 (NCI); Resverastatin phosphate sodium; BPR-OY-007 (National Health Research Institutes); and SSR-250411 (Sanofi)); goserelin; leuprolide; triptolide; homoharringtonine; topotecan; itraconazole; deoxyadenosine; sertraline; pitavastatin; clofazimine; 5-nonyloxytryptamine; vemurafenib; dabrafenib; gefitinib (IRESSA); erlotinib (TARCEVA); cetuximab (ERBITUX); lapatinib (TYKERB); panitumumab (VECTIBIX); vandetanib (CAPRELSA); afatinibBIBW2992; CI-1033/canertinib; neratinib/HKI-272; CP-724714; TAK-285; AST-1306; ARRY334543; ARRY-380; AG-1478; dacomitinib/PF299804; OSI-420/desmethyl erlotinib; AZD8931; AEE726; pelitinib/EKB-569; CUDC-101; WZ8040; WZ4002; WZ3146; AG-490; XI,647; PD153035; 5- azathioprine; 5-aza-2'-deoxycytidine; 17-N-Allylamino-17-Demethoxygeldanamycin (17-AAG); 20-epi-1,25 dihydroxyvitamin D3; 5 ethynyluracil; and BMS-599626.

The patient to be treated with a combination therapy comprising a CD44-modulating polypeptide described herein and an anti-cancer agent described herein may not have been treated with anti-cancer therapy, radiation therapy, or combination thereof prior to the administration the combination therapy. The cancer patient may be treatment naive.

The patient to be treated with a combination therapy comprising a CD44-modulating polypeptide described herein and an anti-cancer agent described herein may have been treated with anti-cancer therapy, radiation therapy, or combination thereof prior to administration of a CD44-modulating polypeptide described herein in combination with an anti-cancer agent described herein. In one example, a patient described herein has been treated with one, two, three, four, five, or more anti-cancer agents. In another example, a patient described herein has been treated with a combination of anti-cancer agents prior to administration of a CD44-modulating polypeptide described herein. In another example, a patient has been treated with radiation therapy (where the patient has optionally been treated as provided above with one or more anti-cancer agents).

A CD44-modulating polypeptide (e.g., SEQ ID NO: 1, SEQ ID NO: 2) can be a first-line therapy. In cases where the patient has received anti-cancer treatment, radiation therapy, or a combination thereof prior to administration with a CD44-modulating polypeptide (e.g., SEQ ID NO: 1, SEQ ID NO: 2), the CD44-modulating polypeptide (e.g., SEQ ID NO: 1, SEQ ID NO: 2) can be a second, third, fourth or more line treatment. In one example, the CD44-modulating peptide can be SEQ ID NO:1 and can be administered as part of a combination with a first administration of an anti-cancer agent (e.g., paclitaxel) described herein. In another example, the CD44-modulating polypeptide can be SEQ ID NO: 1 and can be administered as part of a combination with an anti-cancer agent described herein (e.g., paclitaxel) as a second or third-line or more line therapy. In another example, the CD44-modulating polypeptide can be SEQ ID NO: 1 or SEQ ID NO:2 and can be administered in combination with an anti-cancer agent described herein (e.g., paclitaxel)as a last-line therapy.

A CD44-modulating polypeptide described herein (e.g., SEQ ID NO: 1, SEQ ID NO: 2) can be administered in an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, 200 mg, 250 mg, 300 mg, 350 mg, 400 mg, 450 mg, 500 mg, 550 mg, 600 mg, 650 mg, 700 mg, 750 mg, 800 mg, 900 mg, 1000 mg, 1100 mg, 1200 mg, 1300 mg, 1400 mg, or 1500 mg. A CD44-modulating polypeptide described herein (e.g., SEQ ID NO: 1, SEQ ID NO: 2) can be administered in an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, 200 mg, 250 mg, 300 mg, 350 mg, 400 mg, or 450 mg. A CD44-modulating polypeptide described herein (e.g., SEQ ID NO: 1, SEQ ID NO: 2) can be administered in an amount of about: 100 mg, 125 mg, 150 mg, 175 mg, 200 mg, 250 mg, 300 mg, 350 mg, or 400 mg.

A CD44-modulating polypeptide described herein (e.g., SEQ ID NO: 1, SEQ ID NO: 2) can be administered in an amount of at least about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, 200 mg, 250 mg, 300 mg, 350 mg, 400 mg, 450 mg, 500 mg, 550 mg, 600 mg, 650 mg, 700 mg, 750 mg, 800 mg, 900 mg, 1000 mg, 1100 mg, 1200 mg, 1300 mg, 1400 mg, or 1500 mg. A CD44-modulating polypeptide described herein (e.g., SEQ ID NO: 1, SEQ ID NO: 2) can be administered in an amount of at least about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, 200 mg, 250 mg, 300 mg, 350 mg, 400 mg, or 450 mg. A CD44-modulating polypeptide described herein (e.g., SEQ ID NO: 1, SEQ ID NO: 2) can be administered in an amount of at least about: 100 mg, 125 mg, 150 mg, 175 mg, 200 mg, 250 mg, 300 mg, 350 mg, or 400 mg.

A CD44-modulating polypeptide (e.g., SEQ ID NO: 1, SEQ ID NO: 2) can be administered in an amount from about: 1 mg to about 500 mg: 50 mg to about 500 mg; 100 mg to about 500 mg; 150 mg to about 500 mg; 250 mg to about 500 mg; or about 300 mg to about 500 mg. A CD44-modulating polypeptide (e.g., SEQ ID NO: 1, SEQ ID NO: 2) can be administered in an amount from about: 100 mg to about 200 mg; 100 mg to about 250 mg; 100 mg to about 300 mg; or 100 mg to about 400 mg.

A CD44-modulating polypeptide (e.g., SEQ ID NO: 1, SEQ ID NO: 2) can be administered in an amount from about: 1 mg to about 1500 mg: 50 mg to about 1500 mg; 100 mg to about 1500 mg; 250 mg to about 1500 mg; 500 mg to about 1500 mg; or about 1000 mg to about 1500 mg. A CD44-modulating polypeptide (e.g., SEQ ID NO: 1, SEQ ID NO: 2) can be administered in an amount from about: 100 mg to about 2000 mg; 100 mg to about 3000 mg; 100 mg to about 4000 mg; 100 mg to about 5000 mg; 100 mg to about 6000 mg; 100 mg to about 7000 mg; 100 mg to about 8000 mg; 100 mg to about 9000 mg; or 100 mg to about 10,000 mg. A CD44-modulating polypeptide (e.g., SEQ ID NO: 1, SEQ ID NO: 2) can be administered in an amount from about: 500 mg to about 2000 mg; 500 mg to about 1750 mg; 1000 mg to about 2000 mg; 1000 mg to about 1500 mg; 1200 mg to about 1800 mg; or about 1300 mg to about 1500 mg. A CD44-modulating polypeptide (e.g., SEQ ID NO: 1, SEQ ID NO: 2) can be administered in an amount from about: 1000 mg to 10000 mg; 2000 mg to 10000 mg; 1000 mg to 7500 mg; 1000 mg to 5000 mg; 750 mg to about 4000 mg; or 2000 mg to 5000 mg.

A CD44-modulating polypeptide described herein (e.g., SEQ ID NO: 1, SEQ ID NO: 2) can be administered as described herein in an amount of about: 25 mg/day to about 1500 mg/day; 25 mg/day to about 1200 mg/day; 25 mg/day to about 1000 mg/day; 25 mg/day to about 750 mg/day; 25 mg/day to about 500 mg/day; 25 mg/day to about 300 mg/day; 25 mg/day to about 250 mg/day; 25 mg/day to about 150 mg/day; 50 mg/day to about 1000 mg/day; 50 mg/day to about 750 mg/day; 50 mg/day to about 500 mg/day; 50 mg/day to about 300 mg/day; 50 mg/day to about 250 mg/day; 50 mg/day to about 150 mg/day; 100 mg/day to about 500 mg/day; 100 mg/day to about 300 mg/day; or about 150 mg/day to about 300 mg/day.

The polypeptide of SEQ ID NO: 1 or SEQ ID NO:2 can be administered in an amount of about: 50 mg, 100 mg, 150 mg, 200 mg, 250 mg, or 300 mg. The polypeptide of SEQ ID NO: 1 or SEQ ID NO:2 can be administered as described herein in an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, 200 mg, 250 mg, 300 mg, 350 mg, 400 mg, 450 mg, 500 mg, 550 mg, 600 mg, 650 mg, 700 mg, 750 mg, 800 mg, 900 mg, 1000 mg, 1100 mg, 1200 mg, 1300 mg, 1400 mg, or 1500 mg. The polypeptide of SEQ ID NO: 1 or SEQ ID NO:2 can be administered in an amount of about: 50 mg, 100 mg, 150 mg, 200 mg, 250 mg, or 300 mg. The polypeptide of SEQ ID NO: 1 or SEQ ID NO:2 can be administered in an amount of about: 100 mg, 200 mg, 300 mg, 400 mg, or 500 mg.

The polypeptide of SEQ ID NO: 1 or SEQ ID NO:2 can be administered in an amount of at least about: 50 mg, 100 mg, 150 mg, 200 mg, 250 mg, or 300 mg. The polypeptide of SEQ ID NO: 1 or SEQ ID NO:2 can be administered as described herein in an amount of at least about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, 200 mg, 250 mg, 300 mg, 350 mg, 400 mg, 450 mg, 500 mg, 550 mg, 600 mg, 650 mg, 700 mg, 750 mg, 800 mg, 900 mg, 1000 mg, 1100 mg, 1200 mg, 1300 mg, 1400 mg, or 1500 mg. The polypeptide of SEQ ID NO: 1 or SEQ ID NO:2 can be administered in an amount of at least about: 50 mg, 100 mg, 150 mg, 200 mg, 250 mg, or 300 mg. The polypeptide of SEQ ID NO:1 or SEQ ID NO:2 can be administered in an amount of at least about: 100 mg, 200 mg, 300 mg, 400 mg, or 500 mg.

The polypeptide of SEQ ID NO:1 or SEQ ID NO:2 can be administered as described herein in an amount of about: 25 mg/day to about 1500 mg/day; 25 mg/day to about 1200 mg/day; 25 mg/day to about 1000 mg/day; 25 mg/day to about 750 mg/day; 25 mg/day to about 500 mg/day; 25 mg/day to about 300 mg/day; 25 mg/day to about 250 mg/day; 25 mg/day to about 150 mg/day; 50 mg/day to about 1000 mg/day; 50 mg/day to about 750 mg/day; 50 mg/day to about 500 mg/day; 50 mg/day to about 300 mg/day; 50 mg/day to about 250 mg/day; 50 mg/day to about 150 mg/day; 100 mg/day to about 500 mg/day; 100 mg/day to about 300 mg/day; or about 150 mg/day to about 300 mg/day.

In certain instances it can be useful to administer the CD44-modulating polypeptide (e.g., SEQ ID NO: 1, SEQ ID NO: 2) as an amount relative to the weight of the patient. A polypeptide of SEQ ID NO:1 or SEQ ID NO:2 can be administered as an amount relative to the weight of the patient. A polypeptide of SEQ ID NO:1 or SEQ ID NO:2 can be administered in an amount of about: 0.5 mg/kg, 1 mg/kg, 2 mg/kg, 3 mg/kg, 5 mg/kg, 6 mg/kg, 7 mg/kg, 8 mg/kg, 9 mg/kg, 10 mg/kg, 15 mg/kg, or 20 mg/kg. In certain instances the polypeptide of SEQ ID NO:1 or SEQ ID NO:2 can be administered in an amount of about 0.5 mg/kg to about 20 mg/kg; 0.5 mg/kg to about 10 mg/kg; 0.5 mg/kg to about 7.5 mg/kg; 0.5 mg/kg to about 5 mg/kg; or about 0.5 mg/kg to about 1 mg/kg.

CD44-modulating polypeptides (e.g., SEQ ID NO: 1, SEQ ID NO: 2) can be administered in a treatment regimen that includes administration of the polypeptide in any number of days, weeks, or months and over any period of time, typically until disease lapse, unacceptable toxicity, patient intolerance, or onset of disease symptoms (e.g. relapse or loss of efficacy). CD44-modulating polypeptides described herein (e.g., SEQ ID NO: 1, SEQ ID NO: 2) can be administered at any frequency as described herein such as, for example, once a day (QD), every other day (Q2D), twice daily (BID), once a week (QW), twice weekly (BIW), three times a week (TIW), every other week (Q2W), every three weeks (Q3W), or monthly (QM). In one example a CD44-modulating polypeptide described herein (e.g., SEQ ID NO: 1, SEQ ID NO: 2) can be administered QD for at least 5, 10, 15, 21, 28, 30, 31, 45, 60, 90, 120, 180, or 200 days. In certain instances the CD44-modulating polypeptide (e.g., SEQ ID NO: 1, SEQ ID NO: 2) can be administered QD for at least 30 or 60 days. In another instance the CD44-modulating polypeptide (e.g., SEQ ID NO: 1, SEQ ID NO: 2) can be administered QD for at least 90 or 180 days. In one example the CD44-modulating polypeptide (e.g., SEQ ID NO: 1, SEQ ID NO: 2) can be administered QD until progression of disease or toxicity development.

In another example, a CD44-modulating polypeptide (e.g., SEQ ID NO: 1, SEQ ID NO: 2) can be administered as maintenance therapy before, during, or after treatment with an anti-cancer agent described herein (e.g., paclitaxel). Maintenance therapy refers to long term (e.g., 6 months, or 1, 2, 3, 4, 5, 6 or more years) treatment following a treatment regimen for cancer, such as those described herein, e.g., ovarian cancer, that is intended to keep the cancer in remission. Maintenance therapy can be administered indefinitely following a treatment regimen described herein. In certain instances, CD44-modulating polypeptides (e.g., SEQ ID NO: 1, SEQ ID NO: 2) have little or no toxicity to the patient and continual administration, even after ending a treatment with an anti-cancer agent described herein, radiation therapy described herein, or a combination thereof. In one example, a CD44-modulating polypeptide (e.g., SEQ ID NO: 1, SEQ ID NO: 2) can be administered as a maintenance therapy after the course of a regimen described herein is completed.

In one embodiment, a CD44-modulating polypeptide (e.g., SEQ ID NO: 1, SEQ ID NO: 2) can be administered QD at an amount of about 100 mg to 400 mg. In one embodiment, a CD44-modulating polypeptide (e.g., SEQ ID NO: 1, SEQ ID NO: 2) can be administered QD at an amount of about 1000 mg to 2000 mg. In one embodiment, a CD44-modulating polypeptide (e.g., SEQ ID NO: 1, SEQ ID NO: 2) can be administered QD at an amount of about 150 mg to 300 mg. In one embodiment, a CD44-modulating polypeptide (e.g., SEQ ID NO: 1, SEQ ID NO: 2) can be administered QD at an amount of about 100 mg to about 200 mg or about 250 mg to about 350 mg.

In one example, a CD44-modulating polypeptide (e.g., SEQ ID NO: 1, SEQ ID NO: 2) can be administered BID at an amount of about 100 mg to 400 mg. In one embodiment, a CD44-modulating polypeptide can be administered BID at an amount of about 1000 mg to 2000 mg. In one embodiment, a CD44-modulating polypeptide (e.g., SEQ ID NO: 1, SEQ ID NO: 2) can be administered BID at an amount of about 150 mg to 300 mg. In one embodiment, a CD44-modulating polypeptide (e.g., SEQ ID NO: 1, SEQ ID NO: 2) can be administered BID at an amount of about 100 mg to about 200 mg or about 250 mg to about 350 mg. In one embodiment a CD44-modulating polypeptide (e.g., SEQ ID NO: 1, SEQ ID NO: 2) can be administered BID at an amount of about 150 mg.

In another example, a CD44-modulating polypeptide (e.g., SEQ ID NO: 1, SEQ ID NO: 2) can be administered multiple times a day such that the total amount administered in one day (e.g., about 24 hours) can be about 100 mg to about 400 mg. A CD44-modulating polypeptide (e.g., SEQ ID NO: 1, SEQ ID NO: 2) can be administered multiple times such that the total amount administered in one day can be about 100 mg, 150 mg, 200 mg, 250 mg, 300 mg, or about 350 mg.

In another example, a CD44-modulating polypeptide (e.g., SEQ ID NO: 1, SEQ ID NO: 2) described herein can be administered Q2D. The duration of administration can be the same as described above for QD administration. For example, Q2D administration can be performed for at least 30, 60, 90, 120, or 180 days. In one embodiment, the amount of CD44-modulating polypeptide (e.g., SEQ ID NO: 1, SEQ ID NO: 2) administered Q2D can be equivalent to an amount administered QD. In another embodiment, the amount of CD44-modulating polypeptide (e.g., SEQ ID NO: 1, SEQ ID NO: 2) administered Q2D can be greater than an amount administered QD.

In another example, a CD44-modulating polypeptide (e.g., SEQ ID NO: 1, SEQ ID NO: 2) can be administered once weekly (QW). Once weekly administration can be performed for at least 1, 2, 3, 4, 5, 6, 7, 8 9, 10, 11, 12, 16, 20, or 24 weeks.

QD or BID administration of CD44-modulating polypeptides (e.g., SEQ ID NO: 1, SEQ ID NO: 2) can be performed in a cyclic regimen that includes administration as described above, e.g. 14, 21, or 28 days, and the cycle repeated continually. In one example, QD or BID administration of a CD44-modulating polypeptide (e.g., SEQ ID NO: 1, SEQ ID NO: 2) can be continual without any rest or off period of administration. In another example, administration includes a rest period or off period can be included between each cycle. A rest or off period can be about 1 to 7 days.

In certain instances the effective amount of paclitaxel can be determined as an amount provided in a package insert provided with the agent.

The anti-cancer agent described herein (e.g., paclitaxel) can be administered at any frequency as described herein such as, for example, once a day (QD), twice daily (BID), once a week (QW), twice weekly (BIW), three times a week (TIW), every other week (Q2W), every three weeks (Q3W), or monthly (QM). For example, the anti-cancer agent (e.g., paclitaxel) can be administered BID. An anti-cancer agent (e.g., paclitaxel) can be administered TIW. In certain instances, the anti-cancer agent (e.g., paclitaxel) can be administered 2 to 3 times a week. An anti-cancer agent (e.g., paclitaxel) can be administered QD. An anti-cancer agent (e.g., paclitaxel) can be administered QD for about: 1 day to about 7 days, 1 day to about 14 days, 1 day to about 21 days, 1 day to about 28 days, or daily until disease progression or unacceptable toxicity. Such administration can be performed in cycles such that a 14, 12, or 28 day cycle of treatment can be repeated. When administration is cyclic in nature, a rest period of 1 to 7 days can be included between cycles. The administration of an anti-cancer agent described herein (e.g., paclitaxel) can, in part, depend upon the tolerance of the patient where greater tolerance can allow greater or more frequent administration. Alternatively, where a patient shows poor tolerance to an anti-cancer agent described herein (e.g., paclitaxel), a less amount of the agent or a less frequent dosing can be performed. An anti-cancer agent (e.g., paclitaxel) can be administered in any regimen as described herein.

For example, an anti-cancer agent described herein (e.g., paclitaxel) can be administered at an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, or 200 mg, QD. In one example, an anti-cancer agent described herein (e.g., paclitaxel) includes an agent present at an amount of about: 100 mg, 200 mg, 300 mg, 400 mg, 500 mg, 600 mg, 700 mg, 800 mg, 900 mg, 1000 mg, 1500 mg, 2000 mg, 2500 mg, 3000 mg, 3500 mg, 4000 mg, 4500 mg, 5000 mg, 5500 mg, 6000 mg, 6500 mg, 7000 mg, 8000 mg, 9000 mg, or 10000 mg, QD. For example, an anti-cancer agent described herein (e.g., paclitaxel) can be administered at an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, or 200 mg, BIW. In one example, an anti-cancer agent described herein (e.g., paclitaxel) includes an agent present at an amount of about: 100 mg, 200 mg, 300 mg, 400 mg, 500 mg, 600 mg, 700 mg, 800 mg, 900 mg, 1000 mg, 1500 mg, 2000 mg, 2500 mg, 3000 mg, 3500 mg, 4000 mg, 4500 mg, 5000 mg, 5500 mg, 6000 mg, 6500 mg, 7000 mg, 8000 mg, 9000 mg, or 10000 mg, BIW. For example, an anti-cancer agent described herein (e.g., paclitaxel) can be administered at an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, or 200 mg, TIW. In one example, an anti-cancer agent described herein (e.g., paclitaxel) includes an agent present at an amount of about: 100 mg, 200 mg, 300 mg, 400 mg, 500 mg, 600 mg, 700 mg, 800 mg, 900 mg, 1000 mg, 1500 mg, 2000 mg, 2500 mg, 3000 mg, 3500 mg, 4000 mg, 4500 mg, 5000 mg, 5500 mg, 6000 mg, 6500 mg, 7000 mg, 8000 mg, 9000 mg, or 10000 mg, TIW. For example, an anti-cancer agent described herein (e.g., paclitaxel) can be administered at an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, or 200 mg, QW. In one example, an anti-cancer agent described herein (e.g., paclitaxel) includes an agent present at an amount of about: 100 mg, 200 mg, 300 mg, 400 mg, 500 mg, 600 mg, 700 mg, 800 mg, 900 mg, 1000 mg, 1500 mg, 2000 mg, 2500 mg, 3000 mg, 3500 mg, 4000 mg, 4500 mg, 5000 mg, 5500 mg, 6000 mg, 6500 mg, 7000 mg, 8000 mg, 9000 mg, or 10000 mg, QW. For example, an anti-cancer agent described herein (e.g., paclitaxel) can be administered at an amount of about: 1 mg, 2 mg, 3 mg, 4 mg, 5 mg, 10 mg, 15 mg, 20 mg, 25 mg, 30 mg, 35 mg, 40 mg, 45 mg, 50 mg, 60 mg, 70 mg, 80 mg, 85 mg, 90 mg, 100 mg, 125 mg, 150 mg, 175 mg, or 200 mg, Q2W. In one example, an anti-cancer agent described herein (e.g., paclitaxel) includes an agent present at an amount of about: 100 mg, 200 mg, 300 mg, 400 mg, 500 mg, 600 mg, 700 mg, 800 mg, 900 mg, 1000 mg, 1500 mg, 2000 mg, 2500 mg, 3000 mg, 3500 mg, 4000 mg, 4500 mg, 5000 mg, 5500 mg, 6000 mg, 6500 mg, 7000 mg, 8000 mg, 9000 mg, or 10000 mg, Q2W. Administration of an anti-cancer agent described herein (e.g., paclitaxel) can be continuous. Administration of an anti-cancer agent described herein (e.g., paclitaxel) can be intermittent.

For example, an anti-cancer agent described herein (e.g., paclitaxel) can be administered at an amount of about: 0.0001 mg/kg to about 200 mg/kg, 0.001 mg/kg to about 200 mg/kg, 0.01 mg/kg to about 200 mg/kg, 0.01 mg/kg to about 150 mg/kg, 0.01 mg/kg to about 100 mg/kg, 0.01 mg/kg to about 50 mg/kg, 0.01 mg/kg to about 25 mg/kg, 0.01 mg/kg to about 10 mg/kg, or 0.01 mg/kg to about 5 mg/kg, 0.05 mg/kg to about 200 mg/kg, 0.05 mg/kg to about 150 mg/kg, 0.05 mg/kg to about 100 mg/kg, 0.05 mg/kg to about 50 mg/kg, 0.05 mg/kg to about 25 mg/kg, 0.05 mg/kg to about 10 mg/kg, or 0.05 mg/kg to about 5 mg/kg, 0.5 mg/kg to about 200 mg/kg, 0.5 mg/kg to about 150 mg/kg, 0.5 mg/kg to about 100 mg/kg, 0.5 mg/kg to about 50 mg/kg, 0.5 mg/kg to about 25 mg/kg, 0.5 mg/kg to about 10 mg/kg, or 0.5 mg/kg to about 5 mg/kg, QD. For example, an anti-cancer agent described herein (e.g., paclitaxel) can be administered at an amount of about: 0.0001 mg/kg to about 200 mg/kg, 0.001 mg/kg to about 200 mg/kg, 0.5 mg/kg to about 200 mg/kg, 0.5 mg/kg to about 150 mg/kg, 0.5 mg/kg to about 100 mg/kg, 0.5 mg/kg to about 50 mg/kg, 0.5 mg/kg to about 25 mg/kg, 0.5 mg/kg to about 10 mg/kg, or 0.5 mg/kg to about 5 mg/kg, BIW. For example, an anti-cancer agent described herein (e.g., paclitaxel) can be administered at an amount of about: 0.0001 mg/kg to about 200 mg/kg, 0.001 mg/kg to about 200 mg/kg, 0.5 mg/kg to about 200 mg/kg, 0.5 mg/kg to about 150 mg/kg, 0.5 mg/kg to about 100 mg/kg, 0.5 mg/kg to about 50 mg/kg, 0.5 mg/kg to about 25 mg/kg, 0.5 mg/kg to about 10 mg/kg, or 0.5 mg/kg to about 5 mg/kg, TIW. For example, an anti-cancer agent described herein (e.g., paclitaxel) can be administered at an amount of about: 0.0001 mg/kg to about 200 mg/kg, 0.001 mg/kg to about 200 mg/kg, 0.5 mg/kg to about 200 mg/kg, 0.5 mg/kg to about 150 mg/kg, 0.5 mg/kg to about 100 mg/kg, 0.5 mg/kg to about 50 mg/kg, 0.5 mg/kg to about 25 mg/kg, 0.5 mg/kg to about 10 mg/kg, or 0.5 mg/kg to about 5 mg/kg, QW. For example, an anti-cancer agent described herein (e.g., paclitaxel) can be administered at an amount of about: 0.0001 mg/kg to about 200 mg/kg, 0.001 mg/kg to about 200 mg/kg, 0.5 mg/kg to about 200 mg/kg, 0.5 mg/kg to about 150 mg/kg, 0.5 mg/kg to about 100 mg/kg, 0.5 mg/kg to about 50 mg/kg, 0.5 mg/kg to about 25 mg/kg, 0.5 mg/kg to about 10 mg/kg, or 0.5 mg/kg to about 5 mg/kg, Q2W. Administration of an anti-cancer agent described herein (e.g., paclitaxel) can be continuous. Administration of an anti-cancer agent described herein (e.g., paclitaxel) can be intermittent.

For example, an anti-cancer agent described herein (e.g., paclitaxel) can be administered at an amount of about: 1 mg/kg to about 200 mg/kg, 1 mg/kg to about 150 mg/kg, 1 mg/kg to about 100 mg/kg, 1 mg/kg to about 50 mg/kg, 1 mg/kg to about 25 mg/kg, 1 mg/kg to about 10 mg/kg, or 1 mg/kg to about 5 mg/kg, QD. For example, an anti-cancer agent described herein(e.g., paclitaxel) can be administered at an amount of about: 1 mg/kg to about 200 mg/kg, 1 mg/kg to about 150 mg/kg, 1 mg/kg to about 100 mg/kg, 1 mg/kg to about 50 mg/kg, 1 mg/kg to about 25 mg/kg, 1 mg/kg to about 10 mg/kg, or 1 mg/kg to about 5 mg/kg, BIW. For example, an anti-cancer agent described herein (e.g., paclitaxel) can be administered at an amount of about: 1 mg/kg to about 200 mg/kg, 1 mg/kg to about 150 mg/kg, 1 mg/kg to about 100 mg/kg, 1 mg/kg to about 50 mg/kg, 1 mg/kg to about 25 mg/kg, 1 mg/kg to about 10 mg/kg, or 1 mg/kg to about 5 mg/kg, TIW. For example, an anti-cancer agent described herein (e.g., paclitaxel) can be administered at an amount of about: 1 mg/kg to about 200 mg/kg, 1 mg/kg to about 150 mg/kg, 1 mg/kg to about 100 mg/kg, 1 mg/kg to about 50 mg/kg, 1 mg/kg to about 25 mg/kg, 1 mg/kg to about 10 mg/kg, or 1 mg/kg to about 5 mg/kg, QW. For example, an anti-cancer agent described herein (e.g., paclitaxel) can be administered at an amount of about: 1 mg/kg to about 200 mg/kg, 1 mg/kg to about 150 mg/kg, 1 mg/kg to about 100 mg/kg, 1 mg/kg to about 50 mg/kg, 1 mg/kg to about 25 mg/kg, 1 mg/kg to about 10 mg/kg, or 1 mg/kg to about 5 mg/kg, Q2W.

An anti-cancer agent described herein (e.g., paclitaxel) can be administered as an intravenous infusion over about 10, 20, 30, 40, 50, or 60 or more minutes. An anti-cancer agent described herein (e.g., paclitaxel) can be administered as an intravenous infusion over about 60 minutes according to a regimen and time period set forth above. An anti-cancer agent described herein (e.g., paclitaxel) can be administered as an intravenous infusion according to a package insert.

Dosages of anti-cancer agents described herein (e.g., paclitaxel) can be modified (*e.g.,* increased or decreased dosage) during treatment as set forth herein and understood in the art.

An anti-cancer agent described herein(e.g., paclitaxel) can independently be administered once daily (QD), or divided into multiple daily doses such as twice daily (BID), three times daily (TID), and four times daily as part of a combination therapy described herein. In addition, the administration can be continuous (*i.e.,* daily for consecutive days or every day), intermittent, *e.g.,* in cycles (*i.e.,* including days, weeks, or months of rest without drug). As used herein, the term "daily" is intended to mean that an anti-cancer agent (e.g., paclitaxel) is administered once or more than once each day, for example, for a period of time. The term "monthly" is intended to mean that an anti-cancer agent (e.g., paclitaxel) is administered once a month or about every 4 weeks for an uninterrupted period of time equal to the number of cycles of administration. The term "continuous" is intended to mean that an anti-cancer agent (e.g., paclitaxel) is administered daily for an uninterrupted period of at least 10 days to 52 weeks. The term "intermittent" or "intermittently" as used herein is intended to mean stopping and starting at either regular or irregular intervals. For example, intermittent administration of an anti-cancer agent for use in methods described herein (e.g., paclitaxel) can be administered for one to six days per week, administration in cycles (*e.g*., daily administration for two to eight consecutive weeks, then a rest period with no administration for up to one week), or administration on alternate days. For example, intermittent administration of an anti-cancer agent for use in methods described herein (e.g., paclitaxel) can be administered for once per month, administration in cycles (*e.g*., monthly administration for two to twelve cycles).

In some embodiments, the frequency of administration of an anti-cancer agent (e.g., paclitaxel) can be in the range of about a daily dose to about a monthly dose. In certain embodiments, administration of an anti-cancer agent (e.g., paclitaxel) can be once a day, twice a day, three times a day, four times a day, once every other day, twice a week, once every week, once every two weeks, once every three weeks, or once every four weeks. In one embodiment, a polypeptide of SEQ ID NO: 1 or SEQ ID NO:2 can be administered in combination with an anti-cancer agent (e.g., paclitaxel) that can be administered once a month. In another embodiment, a polypeptide of SEQ ID NO: 1 or SEQ ID NO:2 can be administered in combination with an anti-cancer agent (e.g., paclitaxel) that can be administered twice a month. In yet another embodiment, a polypeptide of SEQ ID NO: 1 or SEQ ID NO:2 can be administered in combination with an anti-cancer agent (e.g., paclitaxel) that can be administered three times a month. In still another embodiment, a polypeptide of SEQ ID NO: 1 or SEQ ID NO:2 can be administered in combination with an anti-cancer agent (e.g., paclitaxel) that can be administered four times a month (*e.g*., weekly). In another embodiment, a polypeptide of SEQ ID NO: 1 or SEQ ID NO:2 can be administered in combination with an anti-cancer agent (e.g., paclitaxel) that can be administered two, three, four, five, or six times a week. In still another embodiment, a polypeptide of SEQ ID NO: 1 or SEQ ID NO:2 can be administered in combination with an anti-cancer agent (e.g., paclitaxel) that can be administered daily.

The polypeptide of SEQ ID NO: 1 or SEQ ID NO:2 can be provided in amounts that are synergistic with the amount of the anti-cancer agent. The term synergistic refers to a combination therapy of a polypeptide of SEQ ID NO:1 or SEQ ID NO:2 and an anti-cancer described herein (e.g., paclitaxel) that can be more effective than the additive effects of each individual therapy or regimen. The polypeptide of SEQ ID NO: 1 or SEQ ID NO:2 can be synergistic the anti-cancer agent (e.g., paclitaxel) where the administration regimen and dosing regimen of the agents provides a synergistic effect.

Combinations described herein that include a CD44-modulating polypeptide described herein and an anti-cancer described herein (e.g., paclitaxel) can be provided as a pharmaceutical composition suitable for administration via any route to a patient described herein including but not limited to: oral, mucosal (*e.g.,* nasal, inhalation, pulmonary, sublingual, vaginal, buccal, or rectal), parenteral (*e.g.,* subcutaneous, intravenous, bolus injection, intramuscular, or intra-arterial), topical *(e.g.,* eye drops or other ophthalmic preparations), transdermal or transcutaneous administration to a patient. The CD44-modulating polypeptide can be a polypeptide of SEQ ID NO:1 or SEQ ID NO:2. In particular embodiments, a CD44-modulating polypeptide described herein can be formulated as a component of a pharmaceutical composition suitable for transdermal delivery. Routes of administration are embodiments not covered by the claims.

Exemplary of dosage forms include: transdermal systems, tablets; caplets; capsules (*e.g.,* gelatin capsules); cachets; lozenges; suppositories; powders; gels; liquid dosage forms suitable for parenteral administration to a patient; and sterile solids (*e.g.,* crystalline or amorphous solids) that can be reconstituted to provide liquid dosage forms suitable for parenteral administration to a patient. In certain embodiments, the CD44-modulating polypeptide described herein can be formulated for parenteral or oral administration. In certain embodiments, the CD44-modulating polypeptide can be formulated for parenteral or transdermal dosing. In another embodiment, the CD44-modulating polypeptide can be formulated for nasal or parenteral administration.

In one example, the CD44-modulating polypeptide can be formulated for transdermal administration. Transdermal administration includes extended/sustained release transdermal devices (e.g., release of the polypeptide for a period of more than about 1 hour or 1 or more days) and immediate release transdermal devices (e.g., release of the polypeptide over a period of time of less than about 1 hour). In certain instances the CD44-modulating polypeptide can be administered using an immediate release transdermal device that releases the CD44-modulating polypeptide over the course of about 0.5, 1, 2, 3, 4, 5, 6, 7, 8, 9, 10, 11, 12, 13, 14, 15, 20, 25, 30, 45, or 60 minutes. For example, the immediate release transdermal device can release the CD44-modulating polypeptide over a period of time of less than about 60 min; less than about 30 min; less than about 20 min, less than about 15 min; less than about 10 min; less than about 5 min; less than about 3 min; less than about 2 min; less than about 1 min; or less than about 0.5 min. In one embodiment, transdermal delivery of a CD44-modulating polypeptide described herein reduces metabolic degradation of the CD44-modulating polypeptide.

In certain instances, transdermal administration can be advantageous for promoting patient compliance because transdermal administration is noninvasive and the administration can be completed in less than a time period set forth above. In such embodiments, the amount of the CD44-modulating polypeptide administered to a patient can be increased by transdermal administration. Patient compliance is often an impetus for failed therapies because fear, pain, and the process of administration can deter patient compliance. Transdermal administration of CD44-modulating polypeptides described herein can increase patient compliance and increase treatment success.

Transdermal administration can include administration of a CD44-modulating polypeptide in a volume of less than about 2 mL (e.g., less than about 2, 1.5, 1, or 0.5 mL) over a period of time provided above. In one example, CD44-modulating polypeptides described herein are administered using a microstructured (e.g., microneedle or hollow point microneedle) transdermal system (3M). Compositions including CD44-modulating polypeptides for transdermal delivery can be formulated as described herein for transdermal delivery. In certain instances, a CD44-modulating polypeptide and an anti-cancer agent can be formulated for transdermal administration.

When CD44-modulating polypeptides described herein are administered transdermally, the transdermal device can provide an extended or sustained release of a CD44-modulating polypeptide. Such transdermal devices can provide transdermal delivery over a time of about 1, 2, 3, 4, 5, 6, 7, or more days. In one example, transdermal delivery occurs over at least 3 to 7 days. In another example, transdermal delivery occurs over at least 15 to 30 days. Systems and devices for transdermal delivery are known and used in the art.

In another embodiment, a CD44-modulating polypeptide can be formulated for intranasal delivery. A CD44-modulating polypeptide can be formulated in a sufficient volume to permit intranasal administration. The volume can be sufficient to allow a plurality of daily administrations to arrive at a total amount of administration of a CD44-modulating polypeptide in an amount described herein. For example, the volume can be sufficient for 1, 2, 3, 4, or more daily administrations intranasal to arrive at an amount of a CD44-modulating polypeptide provided herein.

In one example, the CD44-modulating polypeptide can be formulated for intranasal administration for a total daily dose of about 100 mg to about 400 mg. In another example, the CD44-modulating polypeptide can be formulated for intranasal administration every 2, 4, 6, 8, or 10 hours. Devices for intranasal administration are known in the art. For example, such administration can be performed using atomization and, for example, an atomizer. In one example a CD44-modulatingpolypeptide can be administered at a total amount of about 300 mg per day by intranasal delivery of an amount of about 75 mg every 6 hours.

The CD44-modulating polypeptide described herein and anti-cancer agents described herein can be formulated the same (*e.g.,* both agents formulated for parenteral administration or oral administration). In certain embodiments, a CD44-modulating polypeptide can be formulated for transdermal delivery and an anti-cancer agent can be formulated for either oral or parenteral administration. The CD44-modulating polypeptide described herein and an anti-cancer agent described herein can be formulated in the same dosage form or as separate dosage forms. The CD44-modulating polypeptide can be a polypeptide of SEQ ID NO: 1 or SEQ ID NO:2.

Pharmaceutical compositions and dosage forms described herein typically include one or more excipients. Suitable excipients are well known to those skilled in the art of pharmacy. Whether a particular excipient is suitable for incorporation into a pharmaceutical composition or dosage form depends on a variety of factors such as, for example, the intended route of administration to the patient. Pharmaceutical compositions described herein can include other agents such as stabilizers, lubricants, buffers, and disintegrants that can reduce the rate by which an active ingredient can decompose in a particular formulation. Pharmaceutical compositions described herein can also include other agents such as gelatins, cellulose, thickening/thinning agents, and penetration enhancers (e.g. sulfoxides, ethanol, PEG, oleic acid) that can control the rate an active ingredient can be delivered by, for example, a transdermal system.

Pharmaceutical compositions described herein can in certain instances include additional active agents other than those in the combinations described herein (*e.g.,* an anti-cancer agent such as those described herein) in an amount provided herein.

Anti-cancer agents described herein can be provided in forms convenient to or facilitate their administration to a patient, or for example, according to the formulation provided with a package insert.

The CD44-modulating polypeptide described herein (e.g., SEQ ID NO: 1, SEQ ID NO: 2) or the anti-cancer agent described herein (paclitaxel) can be provided as controlled release pharmaceutical products, which have a goal of improving drug therapy over that achieved by their non-controlled counterparts. Controlled release formulations can extend activity of the drug, reduce dosage frequency, and increase subject compliance. In addition, controlled release formulations can be used to affect the time of onset of action or other characteristics, such as blood levels of the drug, and can thus affect the occurrence of side (*e.g.,* adverse) effects.

The CD44-modulating polypeptide described herein (e.g., SEQ ID NO: 1, SEQ ID NO: 2) and an anti-cancer agent described herein (paclitaxel) can be provided as a pharmaceutical composition. Such pharmaceutical compositions can, as described above, be optionally formulated as a single administration unit or as individual units for administration of each agent. The CD44-modulating polypeptide described herein (e.g., SEQ ID NO: 1, SEQ ID NO: 2) and the anti-cancer agent described herein (paclitaxel) can be provided as part of a kit. Such kits can, for example, improve patient compliance or improve the accuracy or ease of preparation for administering the combination. The kit includes a CD44-modulating polypeptide described herein (e.g., SEQ ID NO: 1, SEQ ID NO: 2) and an anti-cancer agent (paclitaxel) where the polypeptide and anti-cancer agent (paclitaxel) are supplied in a formulation as described herein. The kit can include a package insert or other information (*e.g.,* prescribing information) useful for administration of the combination to a patient in need thereof, such as a cancer patient described herein.

The contents of kits described herein can be provided in sterile form. The kit components can come ready-to-use. The kit and its contents can be provided in a form that can be ready for administration to the subject in need. In such instances, the components of the kit are supplied as a formulation and optionally in an administration device such that administration requires little to no further action by the user. Where kits include administration devices, such devices include devices known and understood by those skilled in the art for routes of administration described herein, such as but not limited to, syringes, pumps, bags, cups, inhalers, droppers, patches, creams, atomizers, or injectors.

The combination therapies described herein can be administered in a regimen. The regimen can be structured to provide therapeutically effective amounts of a CD44-modulating polypeptide described herein (e.g., SEQ ID NO: 1, SEQ ID NO: 2) and the anti-cancer agent described herein (e.g., paclitaxel) over a predetermined period of time (*e.g.,* an administration time). The regimen can be structured to limit or prevent side-effects or undesired complications of each of or any combination of the CD44-modulating polypeptide described herein (e.g., SEQ ID NO: 1, SEQ ID NO: 2) and the anti-cancer agent (e.g., paclitaxel). The regimen can be structured in a manner that results in increased effect for the CD44-modulating polypeptide described herein (e.g., SEQ ID NO: 1, SEQ ID NO: 2) and the anti-cancer agent described herein (e.g., paclitaxel). Regimens useful for treating cancer can include any number of days of administration which can be repeated as necessary, such as those described herein.

Administration periods can be broken by a rest period that includes no administration of at least one therapy. For example, a regimen can include administration periods that include 2, 3, 5, 7, 10, 15, 21, 28, or more days. In one example, a regimen can include transdermal administration of a CD44-modulating polypeptide described herein (e.g., SEQ ID NO: 1, SEQ ID NO: 2). Such transdermal administration can be performed daily (e.g. QD or BID). In another example, a regimen can include parenteral injection of a CD44-modulating polypeptide (e.g., SEQ ID NO: 1, SEQ ID NO: 2) (e.g. pen injector). Such injections can be performed QD, BID, Q2D, Q3D, or QW. In another example, a regimen can include intranasal administration of a CD44-modulating polypeptide as provided herein (e.g., SEQ ID NO: 1, SEQ ID NO: 2). In certain instances a CD44-modulating polypeptide (e.g., SEQ ID NO: 1, SEQ ID NO: 2) can be administered continuously, without a rest period, during the course of a regimen described herein. In further embodiments, a CD44-modulating polypeptide (e.g., SEQ ID NO: 1, SEQ ID NO: 2) can be administered as a maintenance therapy after treatment with a regimen described herein.

The anti-cancer agent (e.g., paclitaxel) can be administered as described herein in a regimen that includes a 7, 14, 21, or 28 day cycle. The cycle can include a rest period between cycles. In one example, paclitaxel can be administered in a cycle of 21 days following by a 7 day rest period. Such cycles can be repeated 2, 3, 4, 5, 6, 7, 8, 9, 10, 11, 12, or more times.

Regimens can include a rest period of at least 1, 2, 3, 5, 7, 10, or more days, where at least one therapy is no longer administered to a patient. The rest period can be determined by, for example, monitoring the reaction of the patient to the drug or by measuring the efficacy of the treatment. A rest period can be applicable to a single therapy, such that only one therapy of the CD44-modulating polypeptide described herein (e.g., SEQ ID NO: 1, SEQ ID NO: 2) or the anti-cancer agent described herein (e.g., paclitaxel) is discontinued in the rest period but the other therapy(ies) are still administered. Rest periods can be applied to all of the therapies administered to the subject such that the subject receives no therapy for a set period of time during the rest period. In certain instances, the CD44-modulating polypeptide described herein (e.g., SEQ ID NO: 1, SEQ ID NO: 2) can be administered daily without interruption.

Regimens described herein for the treatment of cancer using the combinations described herein may be continued until disease progression or unacceptable toxicity.

The anti-cancer agent described herein (paclitaxel) can be administered QD for about 21 days and a CD44-modulating polypeptide described herein (e.g., SEQ ID NO: 1, SEQ ID NO: 2) can be administered QD. For example, an anti-cancer agent described herein (paclitaxel) can be administered QD and a CD44-modulating polypeptide described herein (e.g., SEQ ID NO: 1, SEQ ID NO: 2) can be administered QD via transdermal administration as described herein.

For example, the anti-cancer agent described herein (e.g., paclitaxel) can be administered QD for about 21 days and a CD44-modulating polypeptide described herein (e.g., SEQ ID NO: 1, SEQ ID NO: 2) can be administered BID. For example, an anti-cancer agent described herein can be administered QD and a CD44-modulating polypeptide described herein (e.g., SEQ ID NO: 1, SEQ ID NO: 2) can be administered BID via transdermal administration as described herein.

For example, the anti-cancer agent described herein (e.g., paclitaxel) can be administered or BIW or TIW for about 21 days and a CD44-modulating polypeptide described herein (e.g., SEQ ID NO: 1, SEQ ID NO: 2) can be administered QD. For example, the anti-cancer agent described herein (e.g., paclitaxel) can be administered or BIW or TIW for about 21 days and a CD44-modulating polypeptide described herein (e.g., SEQ ID NO: 1, SEQ ID NO: 2) can be administered QD via transdermal administration as described herein.

For example, the anti-cancer agent described herein (e.g., paclitaxel) described herein can be administered BIW and a CD44-modulating polypeptide described herein (e.g., SEQ ID NO: 1, SEQ ID NO: 2) can be administered QD. For example, the anti-cancer agent described herein (e.g., paclitaxel) can be administered BIW and a CD44-modulating polypeptide described herein (e.g., SEQ ID NO: 1, SEQ ID NO: 2) can be administered QD via transdermal administration as described herein.

For example, the anti-cancer agent described herein (e.g., paclitaxel) can be administered BIW and a CD44-modulating polypeptide described herein (e.g., SEQ ID NO: 1, SEQ ID NO: 2) can be administered BID. For example, the anti-cancer agent (e.g., paclitaxel) described herein can be administered BIW and a CD44-modulating polypeptide described herein (e.g., SEQ ID NO: 1, SEQ ID NO: 2) can be administered BID via transdermal administration as described herein.

For example, the anti-cancer agent described herein (e.g., paclitaxel) can be administered TIW and a CD44-modulating polypeptide described herein (e.g., SEQ ID NO: 1, SEQ ID NO: 2) can be administered QD. For example, the anti-cancer agent (e.g., paclitaxel) described herein can be administered TIW and a CD44-modulating polypeptide described herein (e.g., SEQ ID NO: 1, SEQ ID NO: 2) can be administered QD via transdermal administration as described herein.

For example, the anti-cancer agent described herein (e.g., paclitaxel) can be administered TIW and a CD44-modulating polypeptide described herein (e.g., SEQ ID NO: 1, SEQ ID NO: 2) can be administered BID. For example, the anti-cancer agent (e.g., paclitaxel) described herein can be administered TIW and a CD44-modulating polypeptide described herein (e.g., SEQ ID NO: 1, SEQ ID NO: 2) can be administered BID via transdermal administration as described herein.

For example, the anti-cancer agent described herein (e.g., paclitaxel) can be administered QW and a CD44-modulating polypeptide described herein (e.g., SEQ ID NO: 1, SEQ ID NO: 2) can be administered QD. For example, the anti-cancer agent (e.g., paclitaxel) described herein can be administered QW and a CD44-modulating polypeptide described herein (e.g., SEQ ID NO: 1, SEQ ID NO: 2) can be administered QD via transdermal administration as described herein.

For example, the anti-cancer agent described herein (e.g., paclitaxel) can be administered QW and a CD44-modulating polypeptide described herein (e.g., SEQ ID NO: 1, SEQ ID NO: 2) can be administered BID. For example, the anti-cancer agent (e.g., paclitaxel) described herein can be administered QW and a CD44-modulating polypeptide described herein (e.g., SEQ ID NO: 1, SEQ ID NO: 2) can be administered BID via transdermal administration as described herein.

For example, the anti-cancer agent described herein (e.g., paclitaxel) can be administered Q2W and a CD44-modulating polypeptide described herein (e.g., SEQ ID NO: 1, SEQ ID NO: 2) can be administered QD. For example, the anti-cancer agent (e.g., paclitaxel) described herein can be administered Q2W and a CD44-modulating polypeptide described herein (e.g., SEQ ID NO: 1, SEQ ID NO: 2) can be administered QD via transdermal administration as described herein.

For example, the anti-cancer agent described herein (e.g., paclitaxel) can be administered Q2W and a CD44-modulating polypeptide described herein (e.g., SEQ ID NO: 1, SEQ ID NO: 2) can be administered BID. For example, the anti-cancer agent (e.g., paclitaxel) described herein can be administered Q2W and a CD44-modulating polypeptide described herein (e.g., SEQ ID NO: 1, SEQ ID NO: 2) can be administered BID via transdermal administration as described herein.

For example, the anti-cancer agent described herein (e.g., paclitaxel) can be administered Q3W and a CD44-modulating polypeptide described herein (e.g., SEQ ID NO: 1, SEQ ID NO: 2) can be administered QD. For example, the anti-cancer agent (e.g., paclitaxel) described herein can be administered Q3W and a CD44-modulating polypeptide described herein (e.g., SEQ ID NO: 1, SEQ ID NO: 2) can be administered BID. For example, the anti-cancer agent described herein (e.g., paclitaxel) can be administered Q3W and a CD44-modulating polypeptide described herein (e.g., SEQ ID NO: 1, SEQ ID NO: 2) can be administered via transdermal administration as described herein.

In one embodiment, a CD44-modulating polypeptide (e.g., SEQ ID NO: 1, SEQ ID NO: 2) can be administered in a combination therapy as described herein to patients with diseases and disorders associated with or characterized by, undesired angiogenesis in combination with additional active ingredients, including, but not limited to, anti-cancer drugs, anti-inflammatories, antihistamines, antibiotics, and steroids.

Patients benefitting from the methods described herein can include patients who have been previously treated for cancer but are non-responsive to standard therapies or radiation therapy. In such instances patients may be non-responsive or have developed resistance to anti-cancer treatment(s) or radiation therapy(ies). Patients may have refractory cancer or cancer that is otherwise non-response to at least one anti-cancer therapy or radiation therapy. A patient may also include those who have not previously been treated (e.g. treatment naive) by administering a combination therapy as described herein. Patients can also include those patients who have undergone surgery in an attempt to treat the disease or condition at issue. The methods and combination therapies described herein are equally applicable to patients who have not undergone surgery prior to administration. Patients currently taking agents for treating cancer (e.g., concurrently chemotherapy, immunotherapy, biologics, or hormonal therapy), or undergoing radiation therapy, can benefit from addition of a CD44-modulating polypeptide described herein (e.g., SEQ ID NO: 1, SEQ ID NO: 2) to the treatment regimen.

The methods of treating described herein may be applicable to patients regardless of patient's age, although some diseases or disorders are more common in certain age groups. Patients with certain preconditions, having undergone certain medical procedures, or are currently taking certain therapies, in certain instances, may be excluded from the methods described herein.

Because patients have heterogeneous clinical manifestations and varying clinical outcomes, the treatment given to a patient may vary, depending, in part, on a combination of (1) prognosis, (2) responsiveness to therapy and (3) tolerance to therapy. The present disclosure provides methods of varying treatment using the CD44-modulating polypeptides described herein in combination with an anti-cancer agent.

Examples of cancers include but are not limited to cancers of the bladder, bone, blood, brain, breast, cervix, chest, colon, endrometrium, esophagus, eye, head and neck, kidney, liver, lymph nodes, lung, mouth, ovaries, pancreas, peritoneal, prostate, rectum, stomach, testis, throat, and uterus, e.g., advanced malignancy, amyloidosis, neuroblastoma, meningioma, hemangiopericytoma, multiple brain metastase, glioblastoma multiforms, glioblastoma, brain stem glioma, poor prognosis malignant brain tumor, malignant glioma, recurrent malignant glioma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, Hodgkin's lymphoma, non-Hodgkin's lymphoma, cutaneous T-Cell lymphoma, cutaneous B-Cell lymphoma, diffuse large B-Cell lymphoma, follicular lymphoma, low grade follicular lymphoma, acute myelogenous leukemia, acute lymphocytic leukemia, chronic myelogenous leukemia, chronic lymphocytic leukemia, malignant melanoma, malignant mesothelioma, malignant pleural effusion mesothelioma syndrome, peritoneal carcinoma, primary peritoneal cancer, papillary serous carcinoma, gynecologic sarcoma, soft tissue sarcoma, scleroderma, cutaneous vasculitis, Langerhans cell histiocytosis, leiomyosarcoma, fibrodysplasia ossificans progressive, hormone refractory prostate cancer, resected high-risk soft tissue sarcoma, unrescectable hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone-insensitive prostate cancer, chemotherapy-insensitive prostate cancer, papillary thyroid carcinoma, follicular thyroid carcinoma, medullary thyroid carcinoma, and leiomyoma. The CD44-modulating polypeptide can be a polypeptide of SEQ ID NO: 1 or SEQ ID NO:2. Ovarian cancer is an embodiment covered by the claims. Other cancers are embodiments not covered by the claims.

In one aspect is a method for treating patients with ovarian cancer by administering a CD44-modulating polypeptide described herein in combination with paclitaxel. The CD44-modulating polypeptide can be a polypeptide of SEQ ID NO: 1 or SEQ ID NO:2.

It is understood that modifications which do not substantially affect the activity of the various embodiments of this invention are also included within the definition of the invention provided herein. Accordingly, the following examples are intended to illustrate but not limit the present invention.

### EXAMPLES

### Example 1:

A6 shares sequence homology with the Link Module of CD44. The Link Module of CD44 has been shown to be critical to Hyaluronan (HA) binding and cell migration. When the CD44 Link Module was substituted with a homologous region of higher HA affinity (TSG-6), cells expressing this chimera bound HA, but failed to migrate and were described as tethered. A6 was shown to increase the binding of CD44-expressing SKOV3 cells to HA coated plates. This effect was blocked with the anti-CD44 antibody, IM7. However, neither A6 nor IM7 had any effect on the binding of CD44-nonexpressing A2780 cells to HA coated plates. These results suggest that increasing adhesion may play a role in the antimetastatic activity of the A6 peptide, and again illustrate correlation of A6 activity with CD44 expression. The study further demonstrated that A6 perturbed the binding of the anti-CD44 antibody, DF1485, to CD44-expressing SKOV3 cells. This was reported to be a partial inhibition which did not result from a competition involving either A6 or CD44. Furthermore, the DF1485 antibody did not recognize A6 or inhibit the binding of an anti-A6 antibody to A6. Without being bound by any particular A6 can induce conformational changes in CD44, resulting in either a lowered affinity of the epitope for DF1485, or preventing DF1485 from binding CD44. The binding of A6 to CD44 results in a modulation of CD44-mediated intracellular signaling and establishes a functional relationship between A6 and CD44 in CD44 expressing cells.

CD44 is a complex multifunctional receptor modulating a variety of cellular processes. Studies described herein demonstrate that A6 inhibits the metastatic process in a CD44 dependent manner. Because CD44 is associated with a chemoresistant and radiation resistant phenotype, which is countered by inhibition of CD44 signaling, A6 is a candidate for inhibition of CD44-mediated resistance. A6 may be used in combination with a cytotoxic chemotherapeutic agent such as paclitaxel to inhibit metastases and to render resistant cells sensitive to said agent (e.g,. paclitaxel). Furthermore, due to the positive safety profile documented for A6, there would be a reduced likelihood of compounding toxicity. This safety profile also invites the use of A6 for longer-term maintenance therapy to prevent recurrence stemming from micrometastases surviving first-line standard of care treatment. A6 has demonstrated activity against CD44 expressing tumor cells and CLL cells, and is a candidate for the treatment of malignant disease and hematological malignancy. A6 has demonstrated clinical safety and efficacy, and by targeting CD44 resistant cells to prevent metastases and recurrence, has the possibility of creating a new paradigm for cancer treatment.

*Persistent or Recurrent Ovarian Cancer:* A Phase 2 trial was conducted in patients with persistent or recurrent epithelial ovarian, fallopian tube, or primary peritoneal carcinoma to evaluate A6 in a patient population with a disease burden greater than that presented in the previously described MOR trial. Patients had received one prior platinum-based chemotherapeutic regimen and were allowed to have received one additional cytotoxic regimen for the management of recurrent or persistent disease. Patients received a 150 mg twice daily subcutaneous dose of A6 and continued on treatment until disease progression or unacceptable toxicity. Response criteria were as defined by RECIST. Primary measures of clinical efficacy were objective tumor response and PFS at 6 months compared to a historical Gynecologic Oncology Group (GOG) dataset based on a similar population of patients. Of the 31 eligible patients evaluated, no responses were observed; 6.5% were progression free for at least 6 months; and 36% of evaluable patients achieved stable disease. A6 was well tolerated but had minimal activity in patients with persistent or recurrent epithelial ovarian, fallopian tube, or primary peritoneal carcinoma under the conditions of this trial. Considering the relationship of A6 to CD44 and the relationship of CD44 to resistant and recurrent disease, it would be of interest to follow this study with a combination trial comparing standard-of-care to standard-of-care plus A6 in this difficult population.

### Example 2:

The effect of A6 plus Cisplatin (DDP) or Paclitaxel (PTX) in the B16F10-DsRed Cell Lung Metastasis Model was tested. Cisplatin and lung tumors are embodiments not covered by the claims.

B16F10-DsRed cells were harvested from cells culture. 1 × 10⁶ cells /200 µl were inoculated into DPBS/mouse via bilateral tail vein in 48 female C57BL/6 mice (Charles River).

Mice were randomized into six groups: Grp1 no treatment, Grp2 PTX (10mg/Kg, IP on days 1 and 8), Grp3 DDP (4mg/Kg, IP, day 1, 4 and 8), Grp4 A6 (200mg/Kg, SC, QD), Grp5 A6 plus PTX, Grp6 A6 plus DDP. Animals were weighed before dosing and then twice a week.

Dosing: A6 dosing started immediately after cell inoculation and consecutively for 11 days; DDP/PTX dosing started immediately after inoculation and then on days as above. The study was ended on day 12, and the lungs were harvested to determine metastatic burden.

A6 ± Paclitaxel (PTX). Tumor burden in the lungs was not significantly reduced by A6 in the presence or absence of paclitaxel.

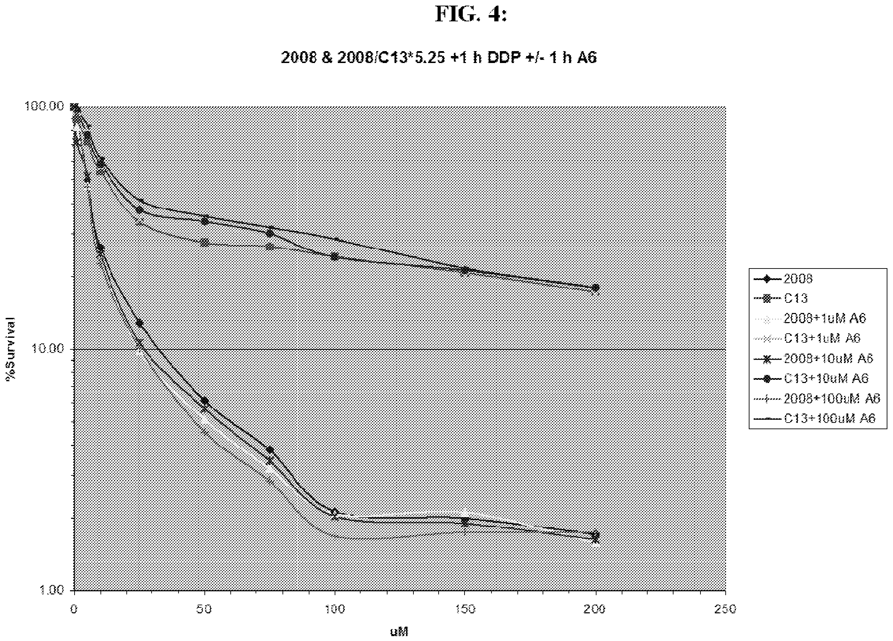

A6 ± Cisplatin (DDP). Tumor burden in the lungs was not significantly reduced by A6 in the presence or absence of cisplatin. (FIG. 4)

Compared to the no treatment group, no treatment significantly reduced the number of tumor nodules in the lung. Furthermore, the addition of A6 did not increase antimetastatic activity of cisplatin or paclitaxel.

### Example 3:

Effect of A6 plus Cisplatin (DDP) or Paclitaxel (PTX) in HEY (DDP sensitive) and HEY/C2 (DDP resistant) cells was tested. Cisplatin is an embodiment not covered by the claims. The cells were plated in 96-well plates as described and incubated overnight. The cells were treating with various doses of DDP / PTX in the absence or presence of 100 µM A6 in culture medium containing 10% FBS and incubated for 72 hours. The CCK-8 assay described herein was performed to access cytotoxicity of DDP / PTX.

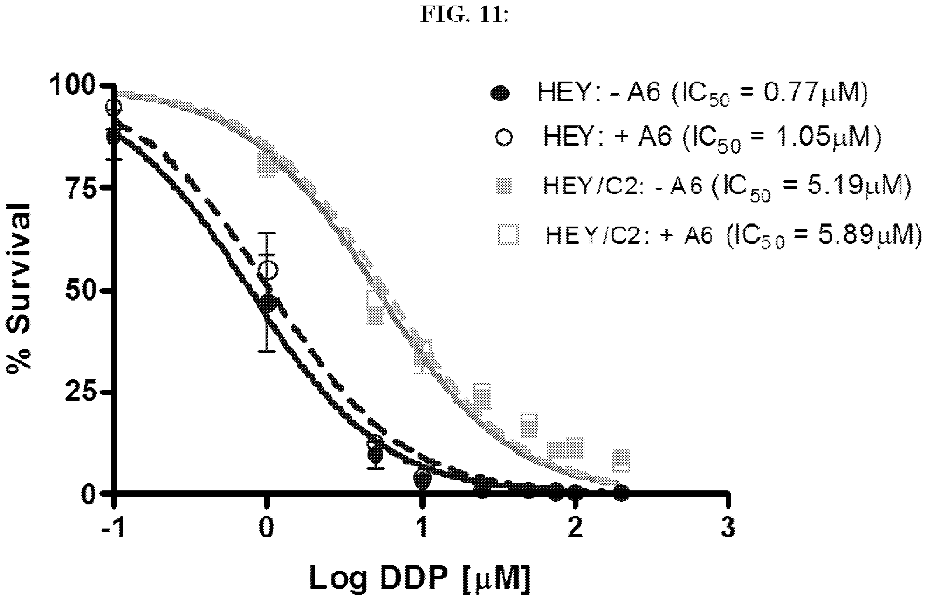

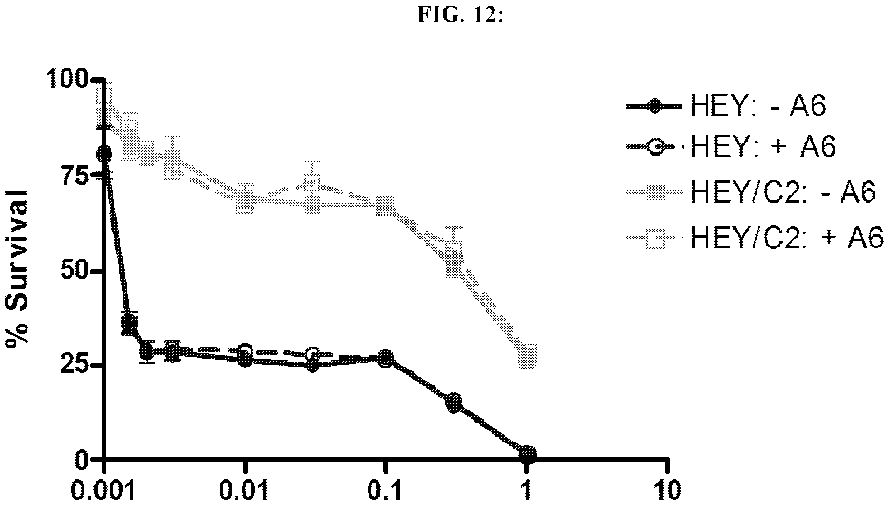

HEY/C2 cells were resistant to DDP with about 7 fold higher IC₅₀ compared to HEY cells. A6 did not affect sensitivity of HEY or HEY/C2 cells to cisplatin. DDP-resistant HEY/C2 cells were also resisted to paclitaxel compared to DDP-sensitive HEY cells. A6 did not affect sensitivity of HEY or HEY/C2 cells to paclitaxel. The dose-response curve appears as biphasic, indicating a possibility of the presence of a relatively resistant cell subpopulation. (See FIGs. 11 and 12).

### Example 4:

A6 was administered in combination with paclitaxel as a compassionate use. The patient (age 65) was diagnosed with breast cancer after mammogram detected a mass. Breast cancer is an embodiment not covered by the claims. A lumpectomy performed was performed resulting in removal of a 19 cm tumor.

The patient was started on adjuvant chemotherapy consisting of Cytoxan (880 mg) and Adriamycin (89 mg) q 3 weeks × 4 followed by radiation therapy to the left breast to a total dose of 4500 cGy.

The patient returned with progressively increasing swelling, redness of skin, tenderness of left breast. Core biopsy revealed diffuse infiltrating ductal carcinoma consistent with recurrence of primary tumor. Breast tissue nearly replaced by tumor - considered rapidly growing and refractory to chemotherapy. Whole body CAT scan and bone scan revealed no identifiable metastases. The patient was administered Taxol, at 268 mg daily for 2 weeks. The patient was started on A6 150 mg/day and continued on this regimen through 9 years of daily treatment. A6 treatment discontinued after 9 years of biannual testing showed no evidence of disease. Patient in complete remission with no clinical evidence of distant metastasis or local recurrence of the cancer.

A second compassionate use patient was administered A6. The patient (age 58) was diagnosed with endometrial cancer with metastases to lymph nodes and lungs. The patient underwent a hysterectomy followed by cisplatin and paclitaxel regimen. Cisplatin and endometrial cancer are embodiments not covered by the claims. The patient started A6 1.5 mg/kg/day and continued on this regimen for 2 years with intermittent radiation therapy to the mediastinum to a total dose of 5,000 cGy. Stable disease was identified and the A6 dose reduced to 0.25-0.5 mg/kg/day. CT scan revealed non-calcified nodules in right lung. CA-125 increased from 25 to 71 U/ml. A6 increased to 2.0 mg/kg/day.

A follow up CT scan revealed no nodules. CA-125 levels reduced to 22 U/ml. Ca-125 remained below 25 U/ml and all subsequent CT scans and PET scans have shown no evidence of disease. A6 treatment discontinued after 10+ years of biannual testing showed no evidence of disease.

## Claims

1. A composition comprising either SEQ ID NO: 1 or SEQ ID NO: 2, and paclitaxel, for use in inhibiting metastasis of ovarian cancer by restoring sensitivity to paclitaxel.

2. The composition for use according to claim 1 further comprising a pharmaceutically acceptable excipient.

## Patentansprüche

1. Pharmazeutische Zusammensetzung, umfassend SEQ ID NO: 1 oder SEQ ID NO: 2, und Paclitaxel, zur Verwendung bei der Hemmung der Metastasierung von Eierstockkrebs durch Wiederherstellung der Empfindlichkeit gegenüber Paclitaxel.

2. Pharmazeutische Zusammensetzung zur Verwendung gemäß Anspruch 1, zusätzlich umfassend einen pharmazeutisch verträglichen Hilfsstoff.

## Revendications

1. Combinaison comprenant soit la SEQ ID NO : 1 soit la SEQ ID NO : 2, et du paclitaxel, pour utilisation dans l'inhibition d'une métastase du cancer des ovaires par le rétablissement de la sensibilité au paclitaxel.

2. Combinaison pour utilisation selon la revendication 1 comprenant en outre un excipient pharmaceutiquement acceptable.
